# EUROPEAN PATENT APPLICATION

(11) **EP 4 428 129 A1**
(43) Date of publication of application: **11.09.2024**
(21) Application number: 22889355.8
(22) Date of filing: 03.11.2022
(51) Int. Cl.: C07D 401/14, A61K 31/4025, A61P 9/00

(54) **SUBSTITUTED PHENYLPROPIONIC ACID DERIVATIVE AND USE THEREOF**

(30) Priority: 03.11.2021 CN 202111293711; 26.08.2022 CN 202211033065
(71) Applicant: Tuojie Biotech (Shanghai) Co., Ltd., Shanghai 201203 (CN)
(72) Inventor: TAN, Liang, Shanghai 201203 (CN); DONG, Yuqiong, Shanghai 201203 (CN); LIU, Min, Shanghai 201203 (CN); LI, Jiao, Shanghai 201203 (CN); LI, Jian, Shanghai 201203 (CN); ZHANG, Zhen, Shanghai 201203 (CN); LIN, Xiaoyan, Shanghai 201203 (CN); LI, Yunfei, Shanghai 201203 (CN)
(74) Representative: Regimbeau
(86) International application number: PCT/CN2022/129479
(87) International publication number: WO 2023/078333

(57) **Abstract**

The disclosure provides a substituted phenylpropionic acid derivative and a use thereof. Specifically, the disclosure provides a compound as shown in formula II or a medicinal salt thereof, which can be used in the preparation of a drug for preventing and/or treating cardiovascular diseases.

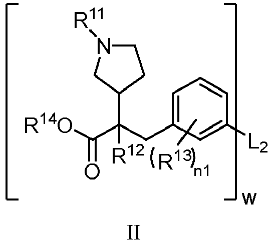

## Description

### TECHNICAL FIELD

The disclosure belongs to the field of pharmaceutics and relates to a substituted phenylpropanoic acid derivative and use thereof.

### BACKGROUND

Lipoprotein(a) [Lp(a)] is a blood lipid particle similar to low-density lipoprotein, primarily composed of a cholesterol ester-rich core and characteristic apolipoprotein(a) [Apo(a)] and characterized by gene polymorphism and long-term stability. It is distributed among people in a skewed manner. Studies have found that elevated levels of Lp(a) are associated with an increased risk of cardiovascular events and related revascularization. While there have been multiple treatment protocols available for elevated levels of LDL-c, reduced levels of HDL-c, and elevated levels of triglycerides, there have been no approved drugs for patients with elevated concentrations of Lp(a).

WO2020247429 reports a class of pyrrolidone-based lipoprotein inhibitors that bind to human Apo(a) protein. These compounds bind to Apo(a) protein to inhibit the binding of LDL particles to Apo(a), thereby reducing Lp(a) levels in the plasma,

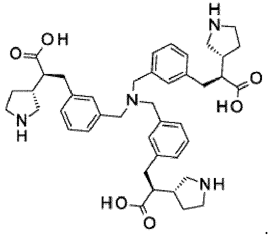

The compounds of the disclosure have not been disclosed in any document, and such compounds demonstrate specific binding to human Apo(a) protein, thereby reducing Lp(a) levels in the plasma.

### SUMMARY

The disclosure provides a compound represented by formula I or a pharmaceutically acceptable salt thereof, 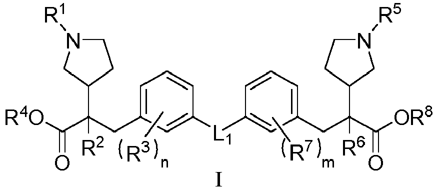
wherein R¹ and R⁵ are independently selected from the group consisting of hydrogen or C₁₋₆ alkyl, wherein the alkyl is optionally substituted with one or more R^{1A}, and each R^{1A} is independently selected from the group consisting of halogen, hydroxy, cyano, or amino; R² and R⁶ are independently selected from the group consisting of hydrogen, deuterium, halogen, or C₁₋₆ alkyl, wherein the alkyl is optionally substituted with one or more R^{2A}, and each R^{2A} is independently selected from the group consisting of halogen, hydroxy, cyano, or amino;
R³ and R⁷ are independently selected from the group consisting of hydrogen, deuterium, halogen, cyano, C₁₋₆ alkyl, or C₁₋₆ alkoxy, wherein the alkyl or alkoxy is optionally substituted with one or more R^{3A}, and each R^{3A} is independently selected from the group consisting of halogen, hydroxy, cyano, or amino;
and R², R³, R⁶, and R⁷ are not simultaneously hydrogen or methyl;
R⁴ and R⁸ are independently selected from the group consisting of hydrogen or C₁₋₆ alkyl; L₁ is selected from the group consisting of -CH₂NHCH₂-, -CH₂NH-, -NH-, -S-, -S(O)-, - S(O)₂-, -O-, -OCH₂-, -OCH₂CH₂O-, -NHSO₂NH-, 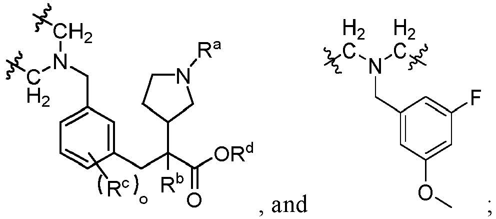
R^{a} is selected from the group consisting of hydrogen or C₁₋₆ alkyl, wherein the alkyl is optionally substituted with one or more R^{4A}, and each R^{4A} is independently selected from the group consisting of halogen, hydroxy, cyano, or amino;
R^{b} is selected from the group consisting of hydrogen, deuterium, halogen, or C₁₋₆ alkyl, wherein the alkyl is optionally substituted with one or more R^{5A}, and each R^{5A} is independently selected from the group consisting of halogen, hydroxy, cyano, or amino; R^{c} is independently selected from the group consisting of hydrogen, deuterium, halogen, cyano, C₁₋₆ alkyl, or C₁₋₆ alkoxy, wherein the alkyl or alkoxy is optionally substituted with one or more R^{6A}, and each R^{6A} is independently selected from the group consisting of halogen, hydroxy, cyano, or amino;
R^{d} is selected from the group consisting of hydrogen or C₁₋₆ alkyl;
n, m, and o are each independently selected from the group consisting of integers between 0-4.

In some embodiments, in the compound of formula I or the pharmaceutically acceptable salt thereof, R² and R⁶ are independently selected from deuterium.

In some embodiments, in the compound of formula I or the pharmaceutically acceptable salt thereof, R² and R⁶ are independently selected from halogen, e.g., fluorine or chlorine. In some embodiments, in the compound of formula I or the pharmaceutically acceptable salt thereof, R² and R⁶ are independently selected from C₁₋₆ alkyl, wherein the alkyl is optionally substituted with one or more R^{2A}, and R^{2A} is as previously defined. In some embodiments, in the compound of formula I or the pharmaceutically acceptable salt thereof, R² and R⁶ are independently selected from the group consisting of methyl, ethyl, or propyl.

In some embodiments, in the compound of formula I or the pharmaceutically acceptable salt thereof, R¹ and R⁵ are independently selected from hydrogen.

In some embodiments, in the compound of formula I or the pharmaceutically acceptable salt thereof, R³ and R⁷ are independently selected from the group consisting of deuterium or C₁₋₆ alkoxy, wherein the alkoxy is optionally substituted with one or more R^{3A}, and R^{3A} is as previously defined.

In some embodiments, in the compound of formula I or the pharmaceutically acceptable salt thereof, R³ and R⁷ are independently selected from halogen, e.g., fluorine or chlorine. In some embodiments, in the compound of formula I or the pharmaceutically acceptable salt thereof, R³ and R⁷ are independently selected from the group consisting of C₁₋₆ alkyl or C₁₋₆ alkoxy, wherein the alkyl or alkoxy is optionally substituted with one or more R^{3A}, and R^{3A} is as previously defined.

Further, in certain embodiments, in the compound of formula I or the pharmaceutically acceptable salt thereof, L₁ is selected from the group consisting of -CH₂NHCH₂-, - CH₂NH-, -NH-, -O-, -OCH₂-, -OCH₂CH₂O-, and -NHSO₂NH-, preferably -CH₂NHCH₂-, -CH₂NH-, -NH-, and -OCH₂CH₂O-.

In some other embodiments, in the compound of formula I or the pharmaceutically acceptable salt thereof, L₁ is selected from 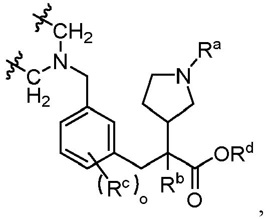 wherein o, R^{a}, R^{b}, and R^{c} are as previously defined.

In some other embodiments, in the compound of formula I or the pharmaceutically acceptable salt thereof, R^{b} is selected from deuterium.

In some other embodiments, in the compound of formula I or the pharmaceutically acceptable salt thereof, R^{b} is selected from halogen, e.g., fluorine or chlorine.

In some other embodiments, in the compound of formula I or the pharmaceutically acceptable salt thereof, R^{b} is selected from C₁₋₆ alkyl, wherein the alkyl is optionally substituted with one or more R^{5A}, and R^{5A} is as previously defined. In some embodiments, in the compound of formula I or the pharmaceutically acceptable salt thereof, R^{b} is independently selected from the group consisting of methyl, ethyl, or propyl.

In some embodiments, in the compound of formula I or the pharmaceutically acceptable salt thereof, R^{a} is selected from hydrogen.

In some embodiments, in the compound of formula I or the pharmaceutically acceptable salt thereof, R^{c} is independently selected from the group consisting of hydrogen or C₁₋₆ alkoxy, wherein the alkoxy is optionally substituted with one or more R^{6A}, and R^{6A} is as previously defined.

In some embodiments, in the compound of formula I or the pharmaceutically acceptable salt thereof, R^{c} is independently selected from halogen, e.g., fluorine or chlorine.

In some embodiments, in the compound of formula I or the pharmaceutically acceptable salt thereof, R^{c} is independently selected from the group consisting of C₁₋₆ alkyl or C₁₋₆ alkoxy, wherein the alkyl or alkoxy is optionally substituted with one or more R^{6A}, and R^{6A} is as previously defined.

In the disclosure, compounds of formula I or pharmaceutically acceptable salts thereof include, but are not limited to:

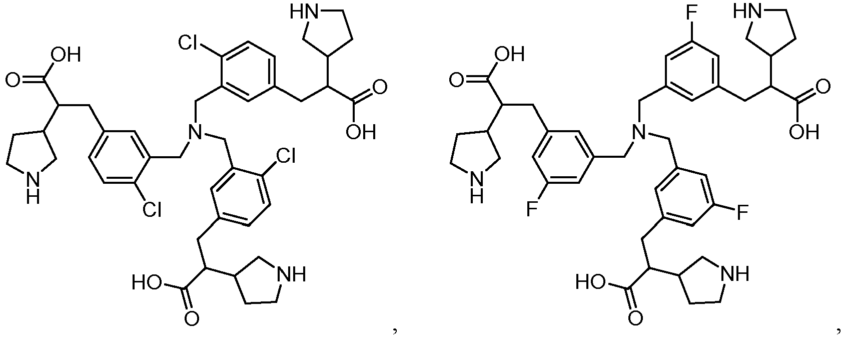

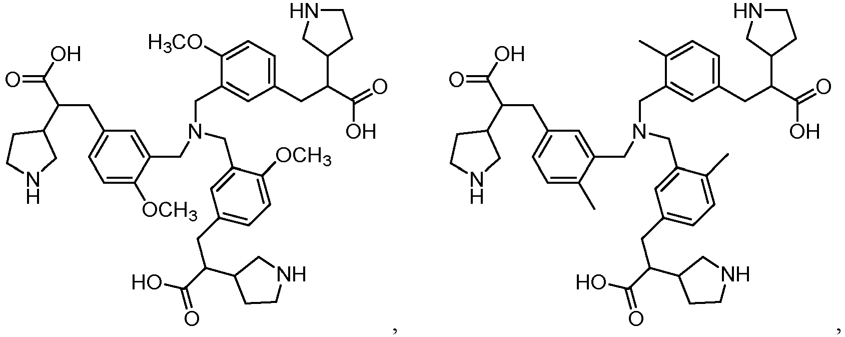

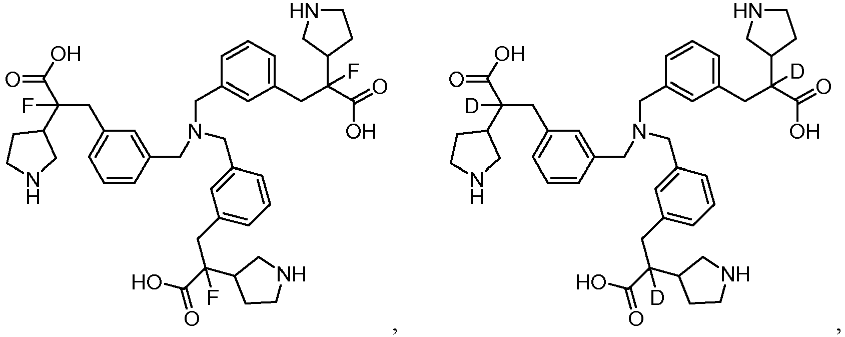

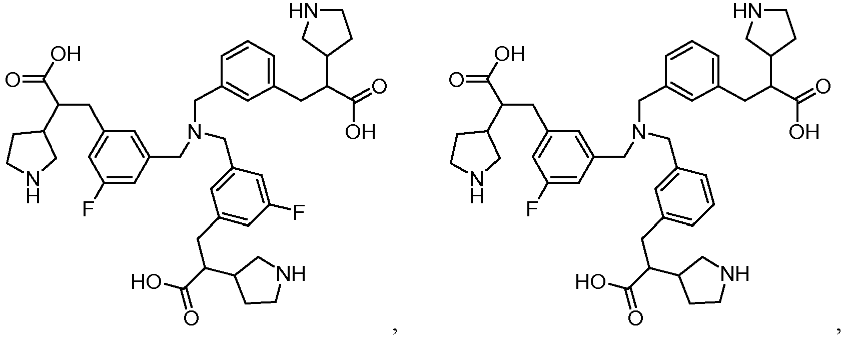

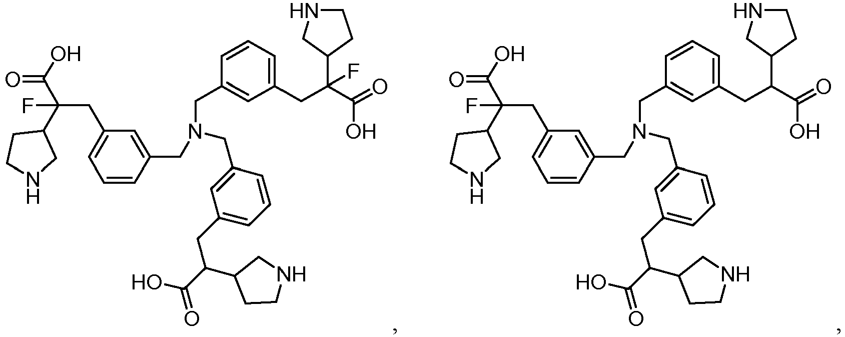

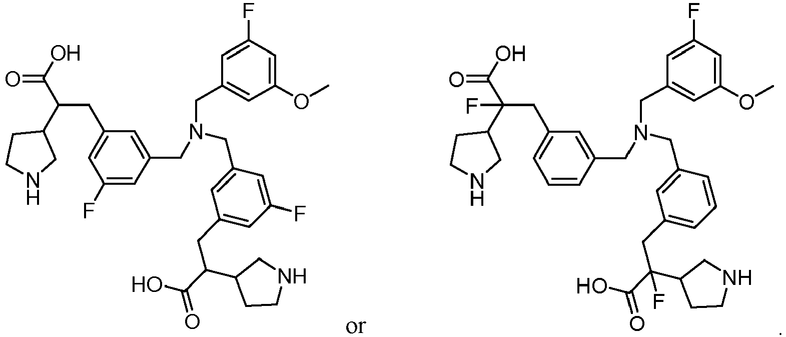

In some embodiments, the compound of formula I or the pharmaceutically acceptable salt thereof is selected from the group consisting of:

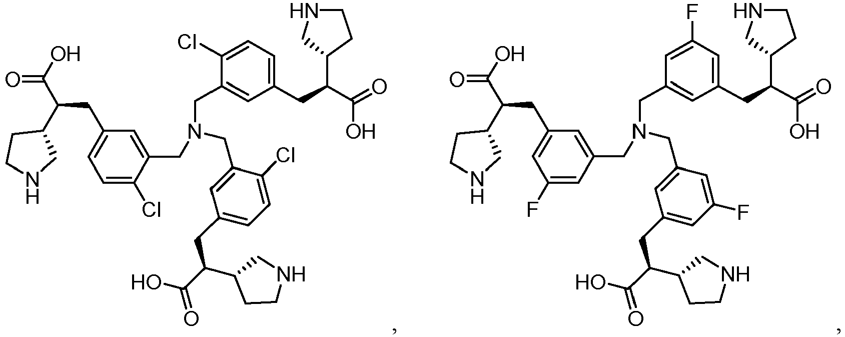

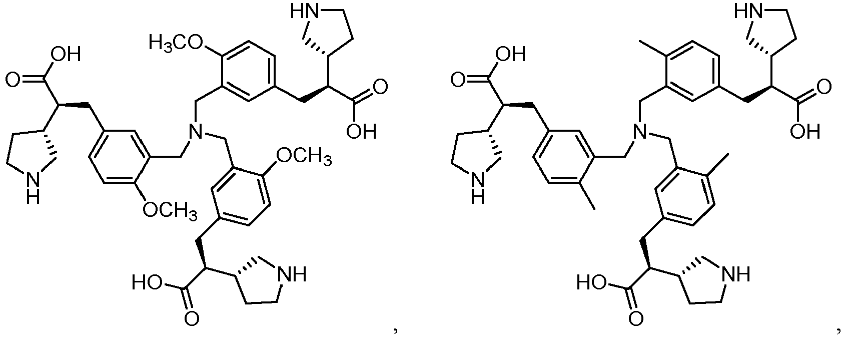

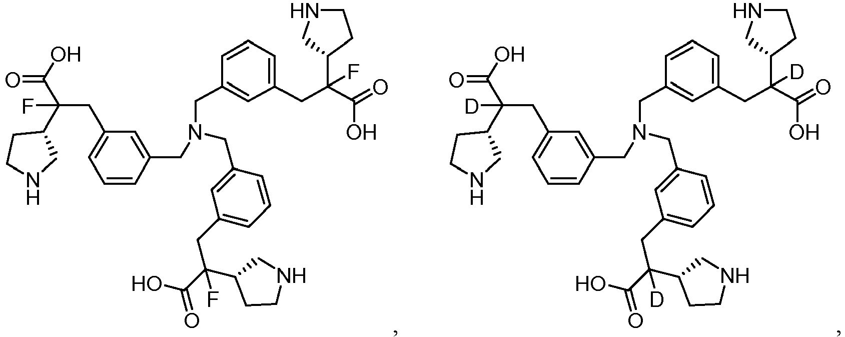

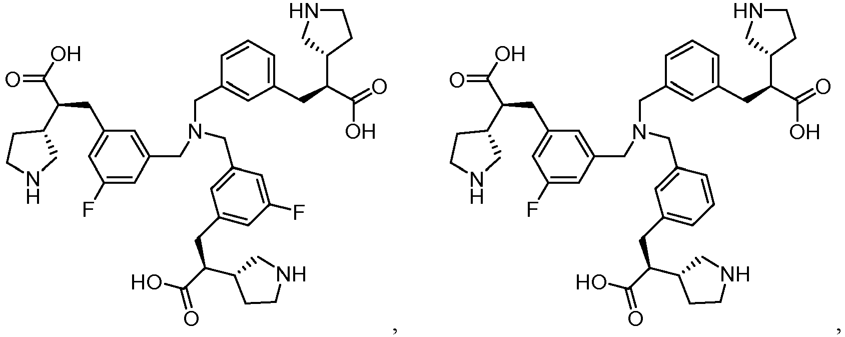

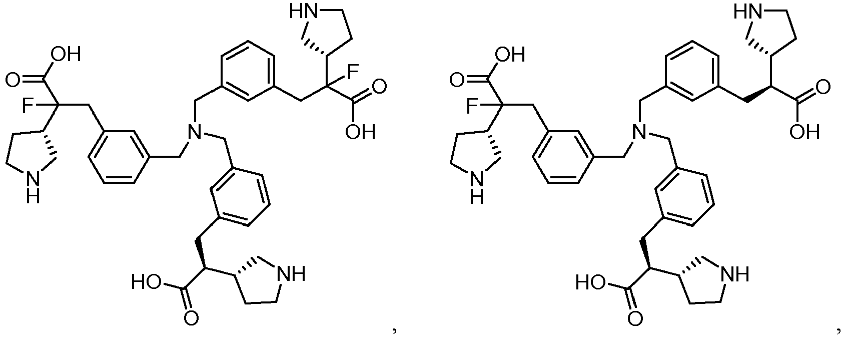

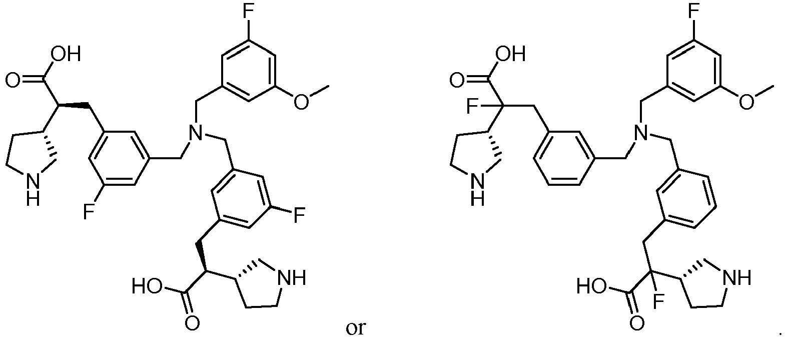

Another aspect of the disclosure also provides a compound represented by formula II or a pharmaceutically acceptable salt thereof, 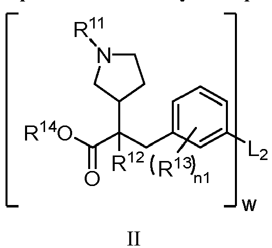
wherein R¹¹ is independently selected from the group consisting of hydrogen or C₁₋₆ alkyl, wherein the alkyl is optionally substituted with one or more R^{11A}, and each R^{11A} is independently selected from the group consisting of halogen, hydroxy, cyano, or amino; R¹² is independently selected from the group consisting of hydrogen, deuterium, halogen, or C₁₋₆ alkyl, wherein the alkyl is optionally substituted with one or more R^{12A}, and each R^{12A} is independently selected from the group consisting of halogen, hydroxy, cyano, or amino;
R¹³ is independently selected from the group consisting of hydrogen, deuterium, halogen, cyano, C₁₋₆ alkyl, or C₁₋₆ alkoxy, wherein the alkyl or alkoxy is optionally substituted with one or more R^{13A}, and each R^{13A} is independently selected from the group consisting of halogen, hydroxy, cyano, or amino;
R¹⁴ is independently selected from the group consisting of hydrogen or C₁₋₆ alkyl;
W is selected from the group consisting of 3 or 4;
L₂ is selected from the group consisting of 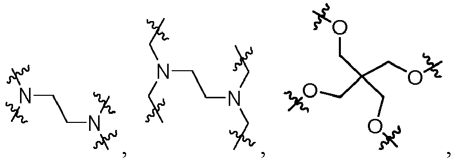 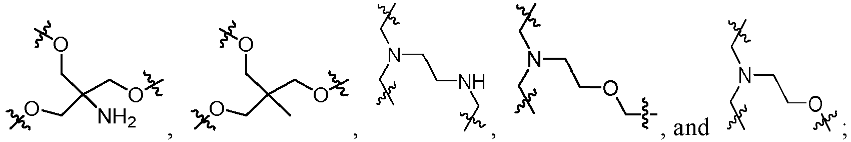
n1 is selected from the group consisting of integers between 0-4.

In some embodiments, in the compound of formula II or the pharmaceutically acceptable salt thereof, R¹² is independently selected from the group consisting of hydrogen or deuterium.

In some embodiments, in the compound of formula II or the pharmaceutically acceptable salt thereof, R¹² is independently selected from halogen, e.g., fluorine or chlorine.

In some embodiments, in the compound of formula II or the pharmaceutically acceptable salt thereof, R¹² is independently selected from C₁₋₆ alkyl, wherein the alkyl is optionally substituted with one or more R^{12A}, and R^{12A} is as previously defined. In some other embodiments, in the compound of formula II or the pharmaceutically acceptable salt thereof, R² and R⁶ are independently selected from the group consisting of methyl, ethyl, or propyl.

In some embodiments, in the compound of formula II or the pharmaceutically acceptable salt thereof, R¹¹ is independently selected from hydrogen.

In some embodiments, in the compound of formula II or the pharmaceutically acceptable salt thereof, R¹³ is independently selected from the group consisting of deuterium or C₁₋₆ alkoxy, wherein the alkoxy is optionally substituted with one or more R^{13A}, and R^{13A} is as previously defined.

In some embodiments, in the compound of formula II or the pharmaceutically acceptable salt thereof, R¹³ is independently selected from halogen, e.g., fluorine or chlorine.

In some other embodiments, in the compound of formula II or the pharmaceutically acceptable salt thereof, L₂ is selected from the group consisting of 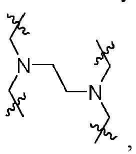 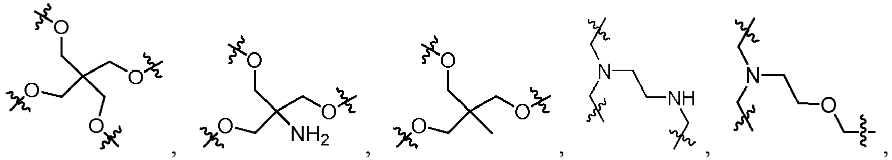 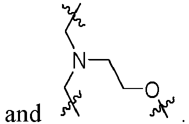 In some other embodiments, in the compound of formula II or the pharmaceutically acceptable salt thereof, L₂ is selected from the group consisting of

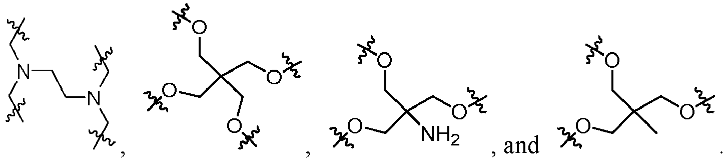

In some other embodiments, in the compound of formula II or the pharmaceutically acceptable salt thereof, L₂ is selected from the group consisting of

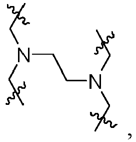

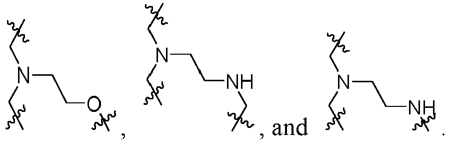

In some other embodiments, in the compound of formula II or the pharmaceutically acceptable salt thereof, L₂ is

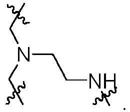

In the disclosure, compounds of formula II or pharmaceutically acceptable salts thereof include, but are not limited to:

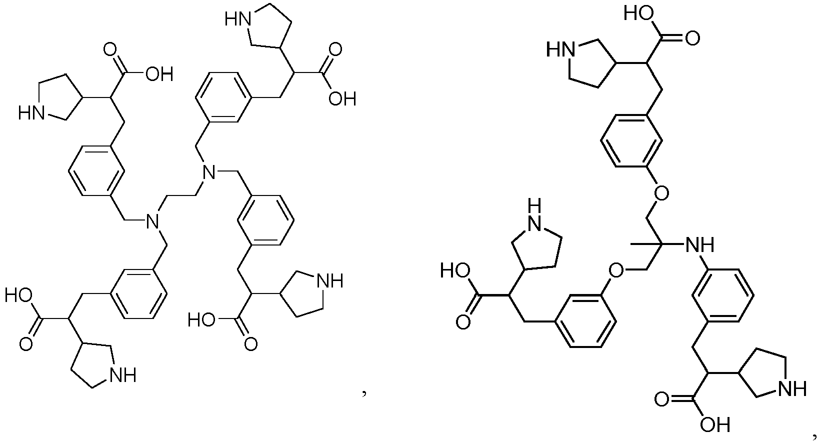

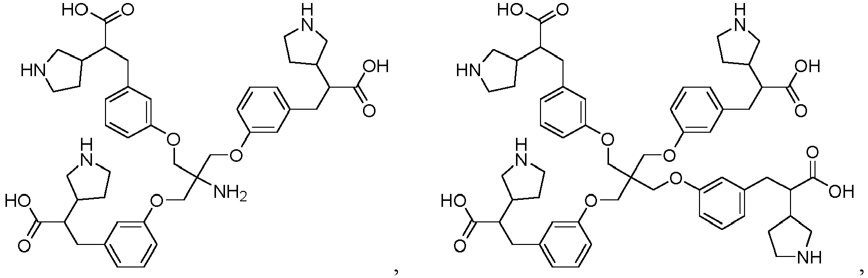

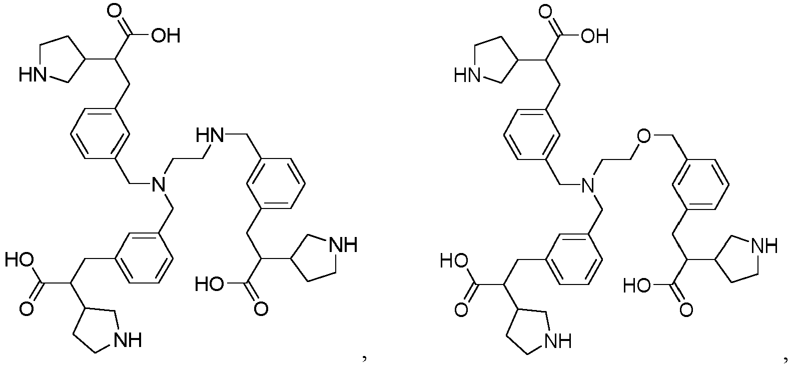

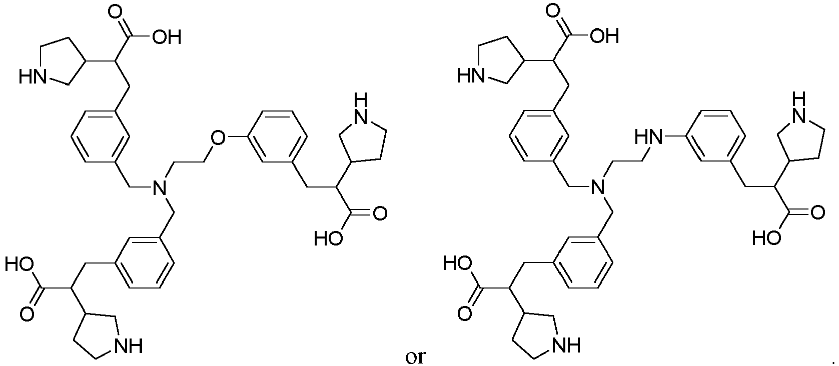

In some embodiments, the compound represented by formula II or the pharmaceutically acceptable salt thereof is selected from the group consisting of:

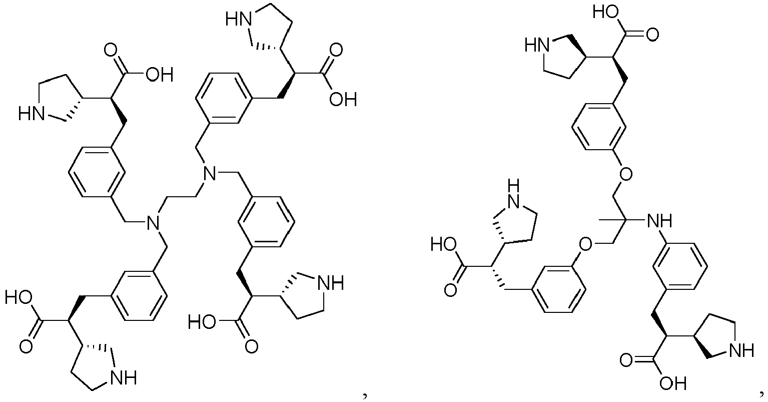

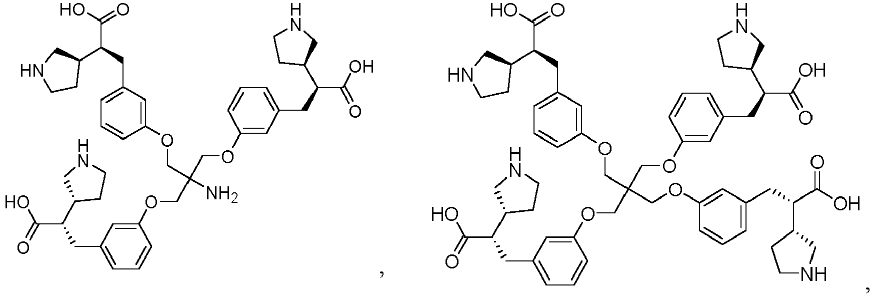

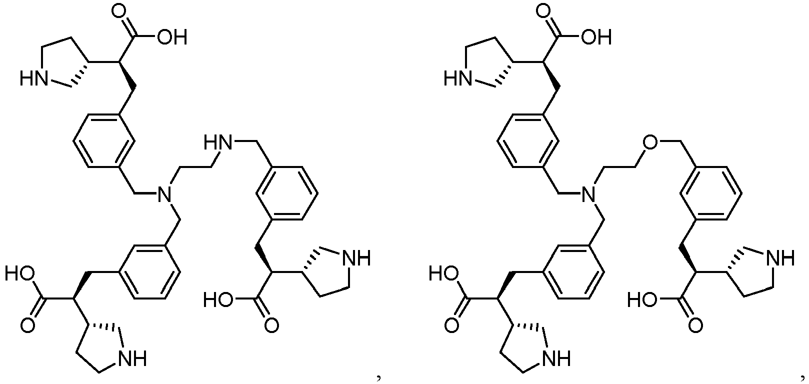

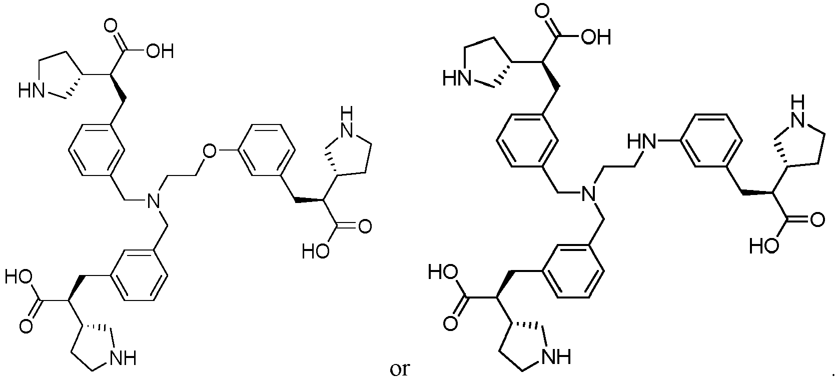

Another aspect of the disclosure also provides a compound represented by formula III or a pharmaceutically acceptable salt thereof, 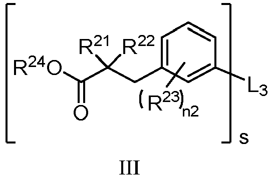
wherein R²¹ and R²² are independently selected from the group consisting of hydrogen, deuterium, halogen, C₁₋₆ alkyl, C₁₋₆ alkoxy, four-membered heterocycloalkyl, and six-membered heterocycloalkyl, wherein the alkyl, alkoxy, four-membered heterocycloalkyl, or six-membered heterocycloalkyl is optionally substituted with one or more R^{21A}, and each R^{21A} is independently selected from the group consisting of hydrogen, deuterium, halogen, or C₁₋₆ alkyl,
or R²¹ and R²² form 3-6 membered heterocycloalkyl with the adjacent carbon atom, wherein the heterocycloalkyl is optionally substituted with one or more R^{22A}, and each R^{22A} is independently selected from the group consisting of deuterium, halogen, and C₁₋₆ alkyl;
R²³ is independently selected from the group consisting of hydrogen, deuterium, halogen, cyano, C₁₋₆ alkyl, or C₁₋₆ alkoxy, wherein the alkyl or alkoxy is optionally substituted with one or more R^{23A}, and each R^{23A} is independently selected from the group consisting of halogen, hydroxy, cyano, or amino;
R²⁴ is independently selected from the group consisting of hydrogen or C₁₋₆ alkyl;
S is selected from the group consisting of integers between 2-4;
L₃ is selected from the group consisting of -CH₂NHCH₂-, -CH₂NH-, -NH-, -S-, -S(O)-, - S(O)₂-, -O-, -OCH₂-, -OCH₂CH₂O-, -NHSO₂NH-, a nitrogen atom, 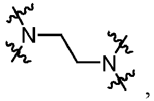 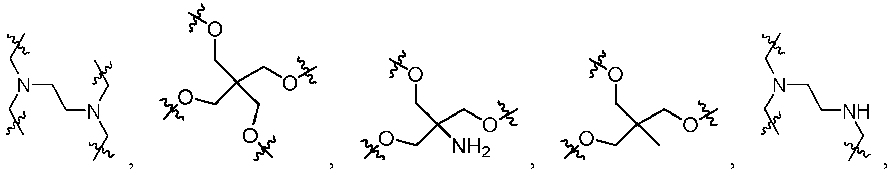 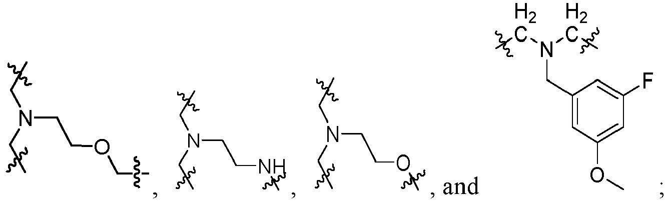
n1 is selected from the group consisting of integers between 0-4.

In some embodiments, in the compound of formula III or the pharmaceutically acceptable salt thereof, R²¹ is independently selected from the group consisting of four-membered heterocycloalkyl or six-membered heterocycloalkyl, and R²² is independently selected from the group consisting of hydrogen, deuterium, halogen, C₁₋₆ alkyl, or C₁₋₆ alkoxy, wherein the alkyl, alkoxy, four-membered heterocycloalkyl, or six-membered heterocycloalkyl is optionally substituted with one or more R^{21A}, and each R^{21A} is independently selected from the group consisting of hydrogen, deuterium, halogen, and C₁₋₆ alkyl.

In some embodiments, in the compound of formula III or the pharmaceutically acceptable salt thereof, R²³ is independently selected from the group consisting of deuterium or C₁₋₆ alkoxy, wherein the alkoxy is optionally substituted with one or more R^{23A}, and R^{23A} is as previously defined.

In some embodiments, in the compound of formula III or the pharmaceutically acceptable salt thereof, R²³ is independently selected from halogen, e.g., fluorine or chlorine.

In some embodiments, the compound of formula III or the pharmaceutically acceptable salt thereof is 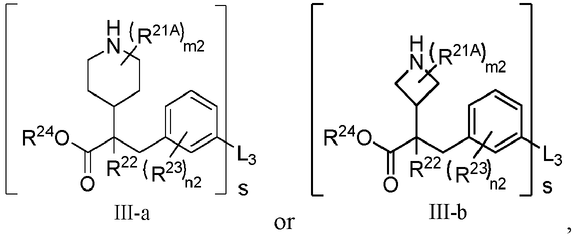 wherein L₃, R²², R²³, R²⁴, R^{21A}, n2, and m2 are as previously defined.

In some other embodiments, in the compound of formula III or the pharmaceutically acceptable salt thereof, L₃ is selected from the group consisting of -CH₂NHCH₂-, - CH₂NH-, -NH-, -S-, -S(O)-, -S(O)₂-, -O-, -OCH₂-, -OCH₂CH₂O-, -NHSO₂NH-, and

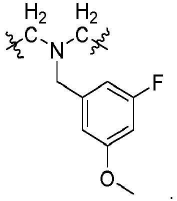

In some other embodiments, the compound of formula III or the pharmaceutically acceptable salt thereof is 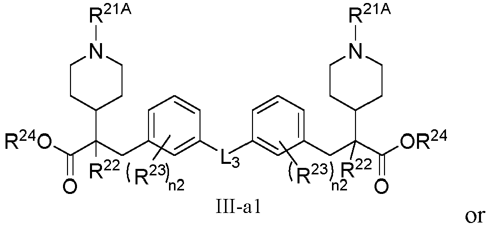 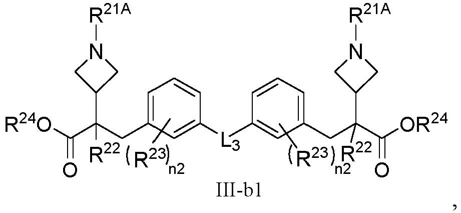 wherein L₃, R²², R²³, R²⁴, R^{21A}, and n2 are as previously defined.

In some embodiments, in the compound of formula III or the pharmaceutically acceptable salt thereof, R²¹ and R²² form 5- or 6-membered heterocycloalkyl with the adjacent carbon atom, wherein the heterocycloalkyl is optionally substituted with one or more R^{22A}, and each R^{22A} is independently selected from the group consisting of deuterium, halogen, and C₁₋₆ alkyl.

In some other embodiments, the compound of formula III or the pharmaceutically acceptable salt thereof is 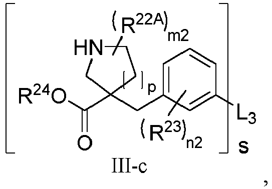 wherein L₃, R²², R²³, R²⁴, R^{22A}, and n2 are as previously defined; p is independently selected from the group consisting of integers between 0-2.

In some embodiments, in the compound of formula III or the pharmaceutically acceptable salt thereof, L₃ is selected from the group consisting of -CH₂NHCH₂-, -CH₂NH-, -NH-, - S-, -S(O)-, -S(O)₂-, -O-, -OCH₂-, -OCH₂CH₂O-, -NHSO₂NH-, and

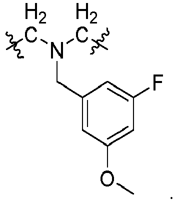

In some embodiments, the compound of formula III or the pharmaceutically acceptable salt thereof is 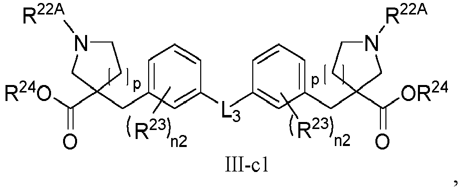 wherein L₃, R²², R²³, R²⁴, R^{21A}, and n2 are as previously defined.

In some embodiments, in the compound of formula III-c or formula III-c1 or the pharmaceutically acceptable salt thereof, p is independently selected from the group consisting of 1 or 2.

In some embodiments, in the compound of formula III-c or formula III-c1 or the pharmaceutically acceptable salt thereof, L₃ is selected from the group consisting of a nitrogen atom,

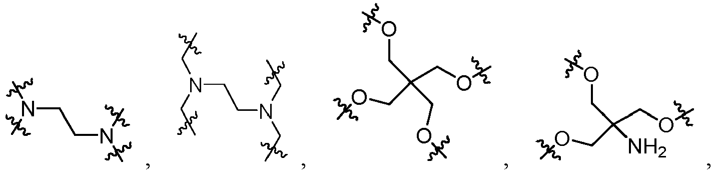

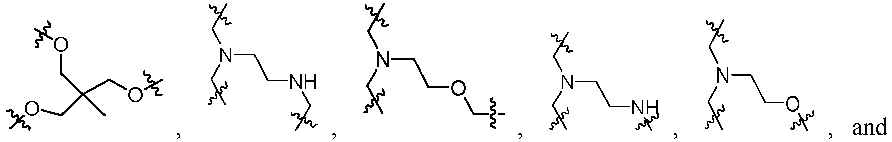

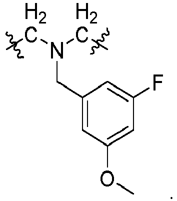

In some embodiments, in the compound of formula III-c or formula III-c1 or the pharmaceutically acceptable salt thereof, L₃ is selected from the group consisting of a nitrogen atom,

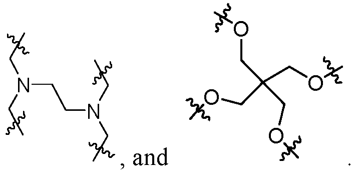

In some embodiments, in the compound of formula III or formula III-c or formula III-c1 or the pharmaceutically acceptable salt thereof, R²⁴ is selected from hydrogen.

In some embodiments, in the compound of formula III or the pharmaceutically acceptable salt thereof, R²³ is selected from the group consisting of hydrogen or C₁₋₆ alkoxy, wherein the alkoxy is optionally substituted with one or more R^{23A}, and R^{23A} is as previously defined.

In some embodiments, in the compound of formula III or the pharmaceutically acceptable salt thereof, R²³ is selected from halogen, e.g., fluorine or chlorine.

In some embodiments, in the compound of formula III or the pharmaceutically acceptable salt thereof, R²³ is selected from the group consisting of C₁₋₆ alkyl or C₁₋₆ alkoxy, wherein the alkyl or alkoxy is optionally substituted with one or more R^{23A}, and R^{23A} is as previously defined.

In some embodiments, in the compound of formula III or the pharmaceutically acceptable salt thereof, R^{21A} is selected from hydrogen.

In the disclosure, compounds represented by formula III or pharmaceutically acceptable salts thereof include, but are not limited to:

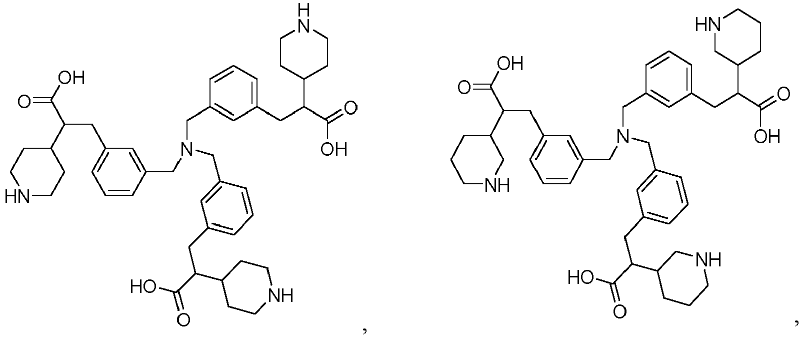

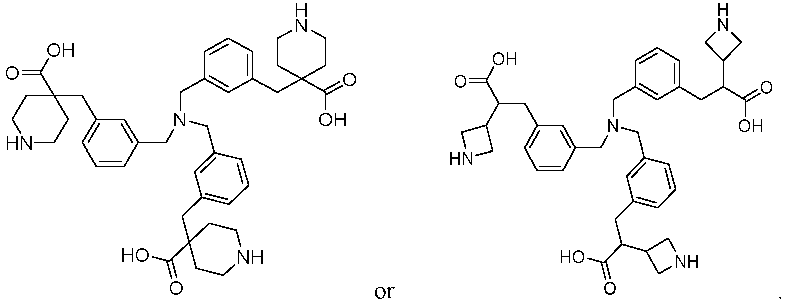

In some embodiments, the compound of formula III or the pharmaceutically acceptable salt thereof is selected from the group consisting of:

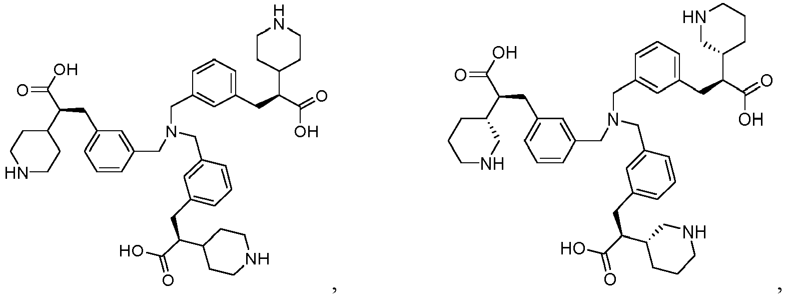

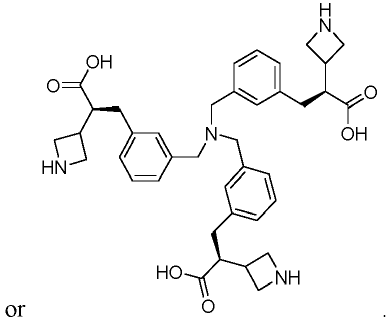

The disclosure also provides isotopically substituted forms of the aforementioned compounds or the pharmaceutically acceptable salts thereof. In some embodiments, the isotopically substituted forms are deuterated forms.

In a competitive binding assay, the *in vitro* binding affinity of the compounds for the expected target human apolipoprotein(a) is tested. The compounds of the disclosure bind to apolipoprotein(a) with a relatively good binding force. In some embodiments, the compounds of the disclosure bind to apolipoprotein(a) with an IC50 value of 0.01 to 500 nM. In some embodiments, the compounds of the disclosure bind to apolipoprotein(a) with an IC50 value of 0.01 to 100 nM. In some embodiments, the compounds of the disclosure bind to apolipoprotein(a) with an IC50 value of 0.01 to 20 nM. In some embodiments, the compounds of the disclosure bind to apolipoprotein(a) with an IC50 value of 0.01 to 20 nM. 0.1 to 30 nM. In some embodiments, the compounds of the disclosure bind to apolipoprotein(a) with an IC₅₀ value of <50 nM.

The disclosure also provides a pharmaceutical composition, comprising a therapeutically effective amount of at least one of the aforementioned compounds represented by formula I or II or III or the pharmaceutically acceptable salts thereof, or the isotopically substituted forms thereof and a pharmaceutically acceptable excipient.

In some embodiments, a unit dose of the pharmaceutical composition is 0.001 mg-1000 mg.

In certain embodiments, the pharmaceutical composition comprises 0.01-99.99% of an aforementioned compound or a pharmaceutically acceptable salt thereof or an isotopically substituted form thereof based on the total weight of the composition. In certain embodiments, the pharmaceutical composition comprises 0.1-99.9% of an aforementioned compound or a pharmaceutically acceptable salt thereof or an isotopically substituted form thereof. In certain embodiments, the pharmaceutical composition comprises 0.5%-99.5% of an aforementioned compound or a pharmaceutically acceptable salt thereof or an isotopically substituted form thereof. In certain embodiments, the pharmaceutical composition comprises 1%-99% of an aforementioned compound or a pharmaceutically acceptable salt thereof or an isotopically substituted form thereof. In certain embodiments, the pharmaceutical composition comprises 2%-98% of an aforementioned compound or a pharmaceutically acceptable salt thereof or an isotopically substituted form thereof.

In certain embodiments, the pharmaceutical composition comprises 0.01%-99.99% of a pharmaceutically acceptable excipient based on the total weight of the composition. In certain embodiments, the pharmaceutical composition comprises 0.1%-99.9% of a pharmaceutically acceptable excipient. In certain embodiments, the pharmaceutical composition comprises 0.5%-99.5% of a pharmaceutically acceptable excipient. In certain embodiments, the pharmaceutical composition comprises 1%-99% of a pharmaceutically acceptable excipient. In certain embodiments, the pharmaceutical composition comprises 2%-98% of a pharmaceutically acceptable excipient.

The disclosure also provides a method for preventing and/or treating a disease or disorder associated with elevated plasma levels of LP(a), by administering to the patient a therapeutically effective amount of the aforementioned compound represented by formula I or II or III or the pharmaceutically acceptable salt thereof or the isotopically substituted form thereof, or the aforementioned pharmaceutical composition.

In some embodiments, the disease or disorder associated with elevated plasma levels of LP(a) is selected from the group consisting of cardiovascular diseases, including but not limited to stroke, hypertensive heart disease, and coronary heart diseases.

The disclosure also provides a method for preventing and/or treating a patient afflicted with a cardiovascular disease, comprising administering to the patient a therapeutically effective amount of the aforementioned compound represented by formula I or formula II or formula III or the pharmaceutically acceptable salt thereof or the isotopically substituted form thereof, or the aforementioned pharmaceutical composition.

The disclosure also provides use of the aforementioned compound represented by formula I or formula II or formula III or the pharmaceutically acceptable salt thereof or the aforementioned pharmaceutical composition in the preparation of a medicament for preventing and/or treating a disease or disorder associated with elevated plasma levels of LP(a). In some embodiments, the disease or disorder associated with elevated plasma levels of LP(a) is selected from the group consisting of cardiovascular diseases, including but not limited to stroke, hypertensive heart disease, and coronary heart diseases.

The disclosure also provides use of the aforementioned compound represented by formula I or formula II or formula III or the pharmaceutically acceptable salt thereof or the aforementioned pharmaceutical composition in the preparation of a medicament for preventing and/or treating a cardiovascular disease.

The pharmaceutically acceptable salts of the compounds described in the disclosure may be selected from the group consisting of inorganic salts or organic salts.

The compounds of the disclosure may exist in specific geometric or stereoisomeric forms. The disclosure contemplates all such compounds, including cis and trans isomers, (-)- and (+)-enantiomers, (R)- and (S)-enantiomers, diastereomers, (D)-isomer, (L)-isomer, and racemic mixtures and other mixtures thereof, such as enantiomerically or diastereomerically enriched mixtures, all of which are within the scope of the disclosure. Additional asymmetric carbon atoms may be present in substituents such as an alkyl group. All such isomers and mixtures thereof are included within the scope of the disclosure. The compounds of the disclosure containing asymmetric carbon atoms can be isolated in optically active pure form or in racemic form. The optically active pure form can be isolated from a racemic mixture or synthesized using chiral starting materials or chiral reagents.

Optically active (R)- and (S)-enantiomers and D- and L-isomers can be prepared by chiral synthesis, chiral reagents, or other conventional techniques. If one enantiomer of a certain compound of the disclosure is desired, it may be prepared by asymmetric synthesis or derivatization with a chiral auxiliary, wherein the resulting mixture of diastereomers is separated and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, followed by resolution of diastereomers by conventional methods known in the art, and the pure enantiomers are obtained by recovery. In addition, separation of enantiomers and diastereomers is generally accomplished by chromatography using a chiral stationary phase, optionally in combination with chemical derivatization (e.g., carbamate formation from amines).

In the chemical structure of the compound of the disclosure, a bond " 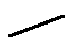 " indicates an unspecified configuration; that is, if chiral isomers exist in the chemical structure, the bond " 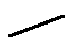 " may be " 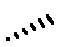 " or " 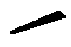 ", or includes both the configurations of " 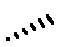 " and " 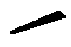 ". In the chemical structures of the compounds of the disclosure, a bond " 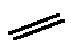 " is not specified with a configuration; that is, they may be in a Z configuration or an E configuration, or contain both configurations.

The compounds and intermediates of the disclosure may also exist in different tautomeric forms, and all such forms are included within the scope of the disclosure. The term "tautomer" or "tautomeric form" refers to structural isomers of different energies that can interconvert via a low energy barrier. For example, proton tautomers (also known as proton transfer tautomers) include interconversion via proton migration, such as keto-enol and imine-enamine, lactam-lactim isomerization. An example of a lactam-lactim equilibrium is present between A and B as shown below.

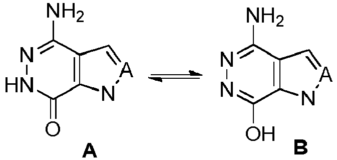

All compounds in the disclosure can be drawn as form A or form B. All tautomeric forms are within the scope of the disclosure. The names of the compounds do not exclude any tautomers.

The disclosure also includes isotopically labeled compounds that are identical to those recited herein but have one or more atoms replaced by an atom having an atomic mass or mass number different from the atomic mass or mass number usually found in nature. Examples of isotopes that can be incorporated into the compounds of the disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, and ³⁶Cl.

Unless otherwise specified, when a position is specifically assigned deuterium (D), the position should be construed as deuterium with an abundance that is at least 1000 times greater than the natural abundance of deuterium (which is 0.015%) (i.e., at least 10% deuterium incorporation). The compounds of examples comprise deuterium having an abundance that is greater than at least 1000 times the natural abundance, at least 2000 times the natural abundance, at least 3000 times the natural abundance, at least 4000 times the natural abundance, at least 5000 times the natural abundance, at least 6000 times the natural abundance, or higher times the natural abundance. The disclosure also includes various deuterated forms of the compound of formula (I). Each available hydrogen atom connected to a carbon atom may be independently replaced by a deuterium atom. Those skilled in the art are able to synthesize the deuterated forms of the compound of formula (I) according to the relevant literature. Commercially available deuterated starting materials can be used in preparing the deuterated forms of the compound of formula (I), or they can be synthesized using conventional techniques with deuterated reagents, including but not limited to deuterated borane, tri-deuterated borane in tetrahydrofuran, deuterated lithium aluminum hydride, deuterated iodoethane, deuterated iodomethane, and the like.

"Optionally" or "optional" means that the event or circumstance subsequently described may, but does not necessarily, occur, and this description includes instances where the event or circumstance occurs or does not occur. For example, "C₁₋₆ alkyl that is optionally substituted with a halogen or cyano" means that the halogen or cyano may, but does not necessarily, exist, and this description includes the instance where the alkyl is substituted with a halogen or cyano and the instance where the alkyl is not substituted with a halogen or cyano.

### Terms and definitions:

"Pharmaceutical composition" refers to a mixture containing one or more of the compounds or the physiologically/pharmaceutically acceptable salts or pro-drugs thereof described herein, and other chemical components, as well as other components, e.g., physiologically/pharmaceutically acceptable carriers and excipients. The pharmaceutical composition is intended to promote administration to an organism and facilitate the absorption of the active ingredient so that it can exert its biological activity.

"Pharmaceutically acceptable excipient" includes, but is not limited to, any adjuvant, carrier, excipient, glidant, sweetener, diluent, preservative, dye/colorant, flavoring agent, surfactant, wetting agent, dispersant, suspending agent, stabilizer, isotonic agent, solvent or emulsifier that has been approved by the drug administration as acceptable for use in humans or livestock animals.

"Effective amount" or "therapeutically effective amount" described in the disclosure includes an amount sufficient to ameliorate or prevent a symptom or disorder of a medical disorder. An effective amount also refers to an amount sufficient to allow or facilitate diagnosis. The effective amount for a particular patient or veterinary subject may vary depending on factors such as the disorder to be treated, the general health of the patient, the method and route and dosage of administration, and the severity of side effects. An effective amount may be the maximum dose or administration regimen to avoid significant side effects or toxic effects.

"Alkyl" refers to a saturated aliphatic hydrocarbon group, including straight-chain and branched-chain groups of 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, various branched isomers thereof, and the like. Alkyl may be substituted or unsubstituted, and when it is substituted, the substituent may be substituted at any accessible point of attachment, preferably one or more of the following groups, independently selected from the group consisting of halogen, hydroxy, cyano, or amino.

The term "heterocycloalkyl" refers to a saturated or partially unsaturated monocyclic or polycyclic hydrocarbon substituent containing 3 to 6 ring atoms. Non-limiting examples of "heterocycloalkyl" include: 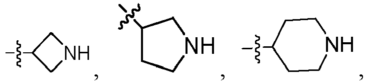 or the like.

Heterocycloalkyl may be optionally substituted or unsubstituted, and when it is substituted, the substituent is preferably one or more of the following groups independently selected from the group consisting of hydrogen, deuterium, halogen, or C₁₋₆ alkyl.

The term "alkoxy" refers to -O-(alkyl), wherein the alkyl is as defined above. Non-limiting examples of alkoxy include: methoxy, ethoxy, propoxy, butoxy, cyclopropyloxy, cyclobutoxy, cyclopentyloxy, and cyclohexyloxy. Alkoxy may be optionally substituted or unsubstituted, and when it is substituted, the substituent is preferably one or more of the following groups independently selected from the group consisting of halogen, hydroxy, cyano, or amino.

The term "heterocyclo" means that the atoms that comprise the ring include not only carbon atoms but also other atoms; the term encompasses heterocycloalkyl and heteroaromatic rings.

The term "hydroxy" refers to the -OH group.

The term "halogen" refers to fluorine, chlorine, bromine, or iodine.

The term "cyano" refers to -CN.

The term "amino" refers to -NH₂.

The term "oxo" refers to the =O substituent.

"Substituted" means that one or more, preferably up to 5, and more preferably 1 to 3, hydrogen atoms in the group are independently substituted with a corresponding number of substituents. It goes without saying that a substituent is only in its possible chemical position, and those skilled in the art will be able to determine (experimentally or theoretically) possible or impossible substitutions without undue effort.

### DETAILED DESCRIPTION

The disclosure is further described below using examples. However, these examples do not limit the scope of the disclosure.

Experimental methods without conditions specified in the examples of the disclosure were generally conducted under conventional conditions, or under conditions recommended by the manufacturers of the starting materials or commercial products. Reagents without specific origins indicated are commercially available conventional reagents.

The structures of the compounds were determined by nuclear magnetic resonance (NMR) spectroscopy and/or mass spectrometry (MS). The NMR shifts (δ) are given in 10⁻⁶ (ppm). The NMR analyses were performed on a Bruker AVANCE-400 nuclear magnetic resonance instrument, with dimethyl sulfoxide-D6 (DMSO-*d₆*), chloroform-D (CDCl₃), and methanol-D4 (CD₃OD) as solvents and tetramethylsilane (TMS) as an internal standard. The spatial configurations of the optical isomers (isomers) of the compounds can be further confirmed by measuring single crystal parameters.

The HPLC analyses were performed on Waters ACQUITY ultra high performance LC, Shimadzu LC-20A systems, Shimadzu LC-2010HT series, or Agilent 1200 LC high performance liquid chromatograph (ACQUITY UPLC BEH C18 1.7 µm 2.1 × 50 mm column, Ultimate XB-C18 3.0 × 150 mm column, or Xtimate C18 2.1 × 30 mm column). The MS analyses were performed on a Waters SQD2 mass spectrometer in positive/negative ion scan mode with a mass scan range of 100-1200.

The Chiral HPLC analyses were performed using a Chiralpak IC-3 100 × 4.6 mm I.D., 3 µm; Chiralpak AD-3 150 × 4.6 mm I.D., 3 µm; Chiralpak AD-3 50 × 4.6 mm I.D., 3 µm; Chiralpak AS-3 150 × 4.6 mm I.D., 3 µm; Chiralpak AS-3 100 × 4.6 mm I.D., 3 µm; ChiralCel OD-3 150 × 4.6 mm I.D., 3 µm; Chiralcel OD-3 100 × 4.6 mm I.D., 3 µm; ChiralCel OJ-H 150 × 4.6 mm I.D., 5 µm; or Chiralcel OJ-3 150 × 4.6 mm I.D., 3 µm column.

The thin-layer chromatography silica gel plates used were Yantai Huanghai HSGF254 or Qingdao GF254 silica gel plates. The silica gel plates used in the thin-layer chromatography (TLC) analyses had a layer thickness of 0.15 mm-0.2 mm, and those used in the thin-layer chromatography separations and purifications had a layer thickness of 0.4 mm-0.5 mm.

The flash column purification system used was Combiflash Rf150 (TELEDYNE ISCO) or Isolara one (Biotage).

In the normal-phase column chromatography steps, a 100-200 mesh, 200-300 mesh, or 300-400 mesh Yantai Huanghai silica gel was generally used as a carrier, or a Changzhou Santai pre-fill ultrapure normal-phase silica gel column (40-63 µm, 60 g, 12 g, 25 g, 40 g, 80 g, or other specifications) was used.

In the reversed-phase column chromatography steps, a Changzhou Santai pre-fill ultrapure C18 silica gel column (20-45 µm, 100 Å, 40 g, 80 g, 120 g, 220 g, or other specifications) was generally used.

The high-pressure column purification system used was Waters AutoP equipped with a Waters XBridge BEH C18 OBD Prep Column, 130 Å, 5 µm, 19 mm × 150 mm or Atlantis T3 OBD Prep Column, 100 Å, 5 µm, 19 mm × 150 mm.

In the preparative chiral chromatography steps, a DAICEL CHIRALPAK IC (250 mm × 30 mm, 10 µm) or Phenomenex-Amylose-1 (250 mm × 30 mm, 5 µm) column was used. The known starting materials in the disclosure may be synthesized by using or following methods known in the art, or may be purchased from companies such as Shanghai Titan Scientific, ABCR GmbH & Co. KG, Acros Organics, Aldrich Chemical Company, Accela ChemBio Inc., and Chembee Chemicals.

In the examples, the reactions can all be performed in an argon atmosphere or a nitrogen atmosphere unless otherwise specified.

The argon atmosphere or nitrogen atmosphere means that the reaction flask is connected to a balloon containing about 1 L of argon or nitrogen gas.

The hydrogen atmosphere means that the reaction flask is connected to a balloon containing about 1 L of hydrogen gas.

The pressurized hydrogenation reactions were performed using a Parr 3916EKX hydrogenator and a Qinglan QL-500 hydrogenator, or an HC2-SS hydrogenator.

The hydrogenation reactions generally involved 3 cycles of vacuumization and hydrogen filling.

The microwave reactions were performed using a CEM Discover-S 908860 microwave reactor.

In the examples, the solutions refer to aqueous solutions unless otherwise specified.

In the examples, the reaction temperature was room temperature, i.e., 20 °C-30 °C, unless otherwise specified.

The monitoring of reaction progress in the examples was performed using thin-layer chromatography (TLC). For the developing solvents used in the reactions, the eluent systems used in the column chromatography purifications, and the developing solvent systems for thin-layer chromatography, the volume ratio of the solvents was adjusted depending on the polarity of a compound, or by adding a small amount of a basic or acidic reagent such as triethylamine and acetic acid.

### Example 1

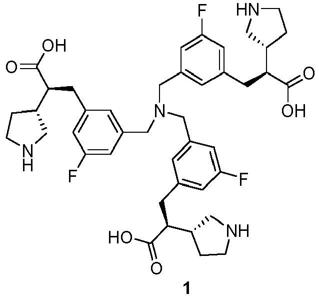

### (2S)-3-(3-{[Bis({3-[(2S)-2-carboxy-2-[(3R)-pyrrolidin-3-yl]ethyl]-5-fluorophenyl}methyl)amino]methyl}-5-fluorophenyl)-2-[(3R)-pyrrolidin-3-yl]propanoic acid (Compound 1)

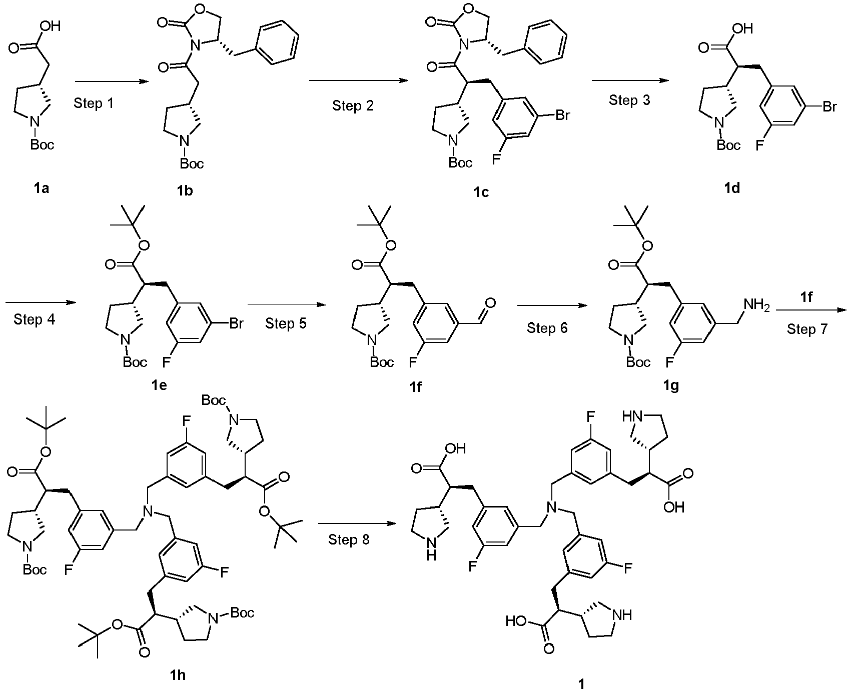

### Step 1: Preparation of tert-butyl (R)-3-(2-((S)-4-benzyl-2-oxooxazolidin-3-yl)-2-oxoethyl)pyrrolidine-1-carboxylate (1b)

The starting material (R)-N-tert-butoxycarbonyl-3-tetrahydropyrroleacetic acid (compound **1a**, 9 g, 39 mmol) was dissolved in anhydrous tetrahydrofuran (100 mL), and the solution was cooled to 0 °C. Triethylamine (13.6 mL, 98 mmol) was added, and the mixture was stirred at 0 °C for 5 min. Pivaloyl chloride (5.8 g, 47.1 mmol) was added, during which the temperature was kept at no more than 10 °C. After 15 min of stirring, lithium chloride (1.9 g, 47.1 mmol) and a solution of (S)-4-benzyl-2-oxazolidinone (6.9 g, 39 mmol) in tetrahydrofuran (100 mL) were added. The reaction mixture was warmed to room temperature and stirred for 24 h. A 2 M aqueous hydrochloric acid solution (500 mL) was added. The organic phase was separated and concentrated, and the residue was purified by silica gel flash column chromatography (petroleum ether/ethyl acetate) to give compound **1b** (12.5 g, yield 82%) as an oily liquid.

MS (ESI): m/z = 411.1 [M+Na]⁺.

### Step 2: Preparation of tert-butyl (R)-3-((S)-1-((S)-4-benzyl-2-oxooxazolidin-3-yl)-3-(3-bromo-5-fluorophenyl)-1-oxopropan-2-yl)pyrrolidine-1-carboxylate (1c)

Compound **1b** (3.5 g, 9 mmol) was dissolved in anhydrous tetrahydrofuran (50 mL), and the solution was cooled to 0 °C in a nitrogen atmosphere. A 1M solution of lithium bis(trimethylsilyl)amide in tetrahydrofuran (10 mL, 10 mmol) was added dropwise. After 15 min of reaction, a solution of 3-fluoro-5-bromobenzyl bromide (2.8 g, 10.8 mmol) in tetrahydrofuran (25 mL) was slowly added dropwise, and the mixture was left to react overnight. After completion of the reaction as monitored by LCMS, the reaction mixture was quenched with saturated NH₄Cl solution (10 mL) and extracted with ethyl acetate. The organic phase was concentrated to give a crude product, and the crude product was purified by reversed-phase flash column chromatography (acetonitrile/water) to give compound **1c** (1.8 g, yield 34.6%).

MS (ESI): m/z = 597.4 [M+Na]⁺.

### Step 3: Preparation of (S)-3-(3-bromo-5-fluorophenyl)-2-((R)-1-(tert-butoxycarbonyl) pyrrolidin-3-yl)propanoic acid (1d)

Compound **1c** (2 g, 3.5 mol) was dissolved in tetrahydrofuran (20 mL) under an ice bath. After 1 M hydrogen peroxide (5 mL) was added, a 0.5 M aqueous lithium hydroxide solution (10 mL) was slowly added dropwise. The reaction was monitored by LCMS. After completion of the reaction, saturated sodium sulfite (10 mL) was added dropwise to quench the reaction, and the mixture was stirred for 5-10 min. A 5 M aqueous sodium hydroxide solution was added dropwise to adjust the pH to >12. Water was added, and extraction was performed with ethyl acetate (100 mL). The aqueous phase was collected, the pH was adjusted to about 3 with a 2 M aqueous hydrochloric acid solution, and extraction was performed with ethyl acetate (100 mL × 2). The organic phase was separated and concentrated to give compound **1d** as a yellow oily liquid. The product was directly used in the next step without purification.

MS (ESI): m/z = 361.7 [M-C(CH₃)₃+H]⁺.

### Step 4: Preparation of tert-butyl (R)-3-((S)-3-(3-bromo-5-fluorophenyl)-1-(tert-butoxy)-1-oxopropan-2-yl)pyrrolidine-1-carboxylate (1e)

Compound **1d** (1.8 g, 4.3 mmol) was dissolved in 2-methyltetrahydrofuran (25 mL), and O-tert-butyl-N,N'-diisopropylisourea (2.17 g, 10.8 mmol) was added. The mixture was heated to 65 °C. After 3 h of reaction, O-tert-butyl-N,N'-diisopropylisourea (862 mg, 4.3 mmol) was added, and the mixture was stirred overnight. After completion of the reaction as monitored by LCMS, the reaction mixture was filtered. The filtrate was concentrated, and the residue was purified by silica gel flash column chromatography (petroleum ether/ethyl acetate) to give compound **1e** (1.3 g, yield 64%).

MS (ESI) m/z = 361.7 [M-2C(CH₃)₃+H]⁺.

### Step 5: Preparation of tert-butyl (R)-3-((S)-1-(tert-butoxy)-3-(3-fluoro-5-formylphenyl)-1-oxopropan-2-yl)pyrrolidine-1-carboxylate (1f)

Compound **1e** (430 mg, 0.9 mmol), palladium acetate (40 mg, 0.2 mmol), 1,4-bis(diphenylphosphino)butane (83 mg, 0.2 mmol), N-formylsaccharin (442 mg, 2.1 mmol), sodium carbonate (270 mg, 2.5 mmol), and triethylsilane (174 mg, 1.5 mmol) were added to a 20 mL microwave reaction tube. In a nitrogen atmosphere, anhydrous N,N-dimethylformamide (6 mL) was added, and the tube was immediately sealed with a plastic screw cap. The mixture was stirred at room temperature for 10 min, and then the reaction mixture was heated to 75 °C and left to react for 16 h. After completion of the reaction as monitored by LCMS, the reaction mixture was cooled to room temperature, centrifuged, and filtered. The filtrate was purified by reversed-phase flash column chromatography (acetonitrile/water) to give compound **1f** (173 mg, yield 50%) as a yellow oil.

MS (ESI): m/z = 444.4 [M+Na]⁺.

### Step 6: Preparation of tert-butyl (R)-3-((S)-3-(3-(aminomethyl)-5-fluorophenyl)-1-(tert-butoxy)-1-oxopropan-2-yl)pyrrolidine-1-carboxylate (1g)

Compound **1f** (100 mg, 0.23 mmol) was dissolved in a 7 M solution of ammonia in methanol (2 mL). One drop of acetic acid was added, and a molecular sieve was added. After 15 min of stirring at room temperature, sodium cyanoborohydride (150 mg, 2.3 mmol) was added, and the tube was sealed and microwaved at 80 °C for 1 h. After completion of the reaction, the reaction mixture was cooled to room temperature, centrifuged, and filtered. The filtrate was purified by reversed-phase flash column chromatography (acetonitrile/water) to give compound **1g** (60 mg, yield 60%) as a yellow oil.

MS (ESI): m/z = 423.8 [M+H]⁺.

### Step 7: Preparation of tert-butyl (3R)-3-[(2S)-3-(3-{[bis({3-[(2S)-3-(tert-butoxy)-2-[(3R)-1-[(tert-butoxy)carbonyl]pyrrolidin-3-yl]-3-propionyl]-5-fluorophenyl }methyl)amino]methyl} -5-fluorophenyl)-1-(tert-butoxy)-1 -oxopropan-2-yl]pyrrolidine-1-carboxylate (1h)

Compound **1g** (60 mg, 0.14 mmol) was dissolved in absolute methanol (8 mL), and compound **1f** (180 mg, 0.412 mmol) was added. At room temperature, 1 drop of acetic acid was added, and a molecular sieve was added. After 15 min of stirring, sodium cyanoborohydride (100 mg, 1.4 mmol) was added, and the mixture was heated to 75 °C. After 4 h of reaction, the mixture was left to react overnight at room temperature. After completion of the reaction as monitored by LCMS, a small amount of DMF was added and dissolved. The mixture was centrifuged and filtered. The filtrate was purified by reversed-phase flash column chromatography (acetonitrile/water) to give compound **1h** (130 mg, yield 74.2%) as a white solid.

MS (ESI): m/z = 1233.9 [M+H]⁺.

### Step 8: Preparation of (2S)-3-(3-{[bis({3-[(2S)-2-carboxy-2-[(3R)-pyrrolidin-3-yl]ethyl]-5-fluorophenyl}methyl)amino]methyl}-5-fluorophenyl)-2-[(3R)-pyrrolidin-3-yl]propanoic acid (1)

Compound **1h** (130 mg, 0.105 mmol) was dissolved in anhydrous dioxane (1 mL), and a 4 M solution of hydrogen chloride in dioxane (3 mL) was added. The mixture was left to react at room temperature. After completion of the reaction as monitored by LCMS, the reaction mixture was concentrated, and the crude product was purified by preparative reversed-phase chromatography (acetonitrile/water + 1% formic acid) to give the title compound **1** (45 mg, yield 55.8%).

MS (ESI): m/z = 765.3 [M+H]⁺.

¹H NMR (400 MHz, D₂O) *δ* 8.45 (s, 1H), 7.10 (d, *J* = 9.7 Hz, 3H), 7.06 - 7.00 (m, 6H), 4.27 (s, 6H), 3.64 - 3.54 (m, 3H), 3.49 - 3.38 (m, 3H), 3.32 - 3.22 (m, 3H), 3.08 - 2.99 (m, 3H), 2.88 - 2.73 (m, 6H), 2.56 - 2.42 (m, 6H), 2.20 - 2.08 (m, 3H), 1.78 (d, *J =* 9.6 Hz, 3H).

### Example 2

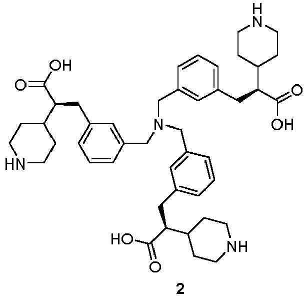

### (2S)-3-(3-{[Bis({3-[(2S)-2-carboxy-2-(piperidin-4-yl)ethyl]phenyl}methyl)amino] methyl }phenyl)-2-(piperidin-4-yl)propanoic acid (2)

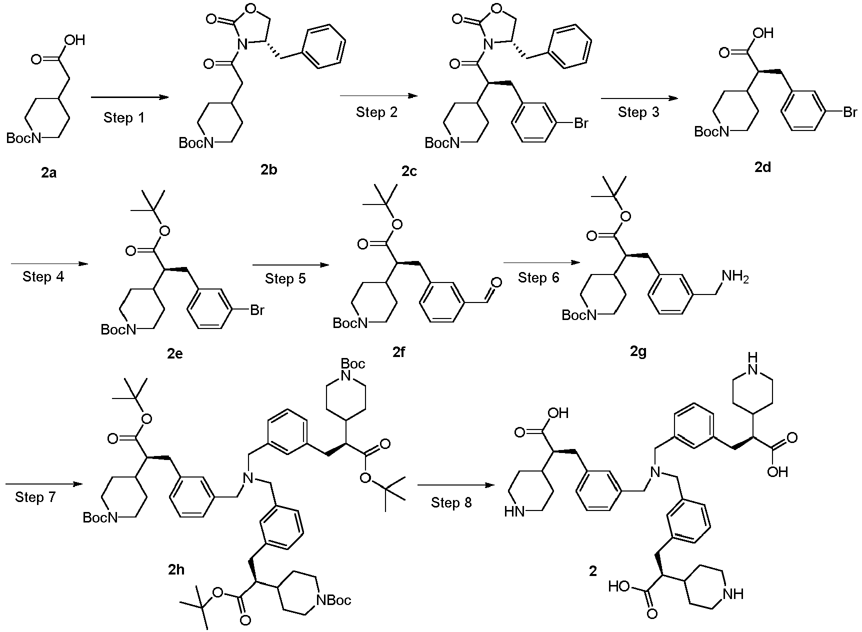

### Step 1: Preparation of tert-butyl (S)-4-(2-(4-benzyl-2-oxooxazolidin-3-yl)-2-oxoethyl)piperidine-1-carboxylate (2b)

The starting material 1-tert-butoxycarbonyl-4-piperidineacetic acid (compound **2a,** 5 g, 21 mmol) was dissolved in anhydrous tetrahydrofuran (50 mL), and the solution was cooled to 0 °C. Triethylamine (7.5 mL, 51 mmol) was added, and the mixture was stirred at 0 °C for 5 min. Pivaloyl chloride (3.0 g, 24 mmol) was added, during which the temperature was kept at no more than 10 °C. After 15 min of stirring, lithium chloride (1.0 g, 24 mmol) and a solution of (S)-4-benzyl-2-oxazolidinone (3.6 g, 22 mmol) in tetrahydrofuran (50 mL) were added. The reaction mixture was warmed to room temperature and stirred for 24 h. A 2 M aqueous hydrochloric acid solution (250 mL) was added. The organic phase was separated and concentrated, and the residue was purified by silica gel flash column chromatography (petroleum ether/ethyl acetate) to give compound **2b** (8 g, yield 96.7%) as an oily liquid.

MS (ESI): m/z = 425.6 [M+Na]⁺.

### Step 2: Preparation of tert-butyl 4-((S)-1-((S)-4-benzyl-2-oxooxazolidin-3-yl)-3-(3-bromophenyl)-1-oxopropan-2-yl)piperidine-1-carboxylate (2e)

Compound **2b** (5 g, 12 mmol) was dissolved in anhydrous tetrahydrofuran (80 mL), and the solution was cooled to 0 °C in a nitrogen atmosphere.

A 1M solution of lithium bis(trimethylsilyl)amide in tetrahydrofuran (15 mL, 15 mmol) was added dropwise. After 15 min of reaction, a solution of 3-bromobenzyl bromide (3 g, 12 mmol) in tetrahydrofuran (15 mL) was slowly added dropwise, and the mixture was left to react overnight. After completion of the reaction as monitored by LCMS, the reaction mixture was quenched with saturated NH₄Cl solution (15 mL) and extracted with ethyl acetate. The organic phase was concentrated to give a crude product, and the crude product was purified by reversed-phase flash column chromatography (acetonitrile/water) to give compound **2c** (1.8 g, yield 25.3%).

MS (ESI): m/z = 593.5 [M+Na]⁺.

### Step 3: Preparation of (S)-3-(3-bromophenyl)-2-(1-(tert-butoxycarbonyl)piperidin-4-yl)propanoic acid (2d)

Compound **2c** (1.5 g, 2.6 mol) was dissolved in tetrahydrofuran (15 mL) under an ice bath. After 1 M hydrogen peroxide (3.5 mL) was added, a 0.5 M aqueous lithium hydroxide solution (7 mL) was slowly added dropwise. The reaction was monitored by LCMS. After completion of the reaction, saturated sodium sulfite (8 mL) was added dropwise to quench the reaction, and the mixture was stirred for 5-10 min. A 5 M aqueous sodium hydroxide solution was added dropwise to adjust the pH to >12. Water was added, and extraction was performed with ethyl acetate (80 mL). The aqueous phase was collected, the pH was adjusted to about 3 with a 2 M aqueous hydrochloric acid solution, and extraction was performed with ethyl acetate (80 mL × 2). The organic phase was separated and concentrated to give compound **2d** as a yellow oily liquid. The product was directly used in the next step without purification.

MS (ESI): m/z = 358.7 [M-C(CH₃)₃+H]⁺.

### Step 4: Preparation of tert-butyl (S)-4-(3-(3-bromophenyl)-1-(tert-butoxy)-1-oxopropan-2-yl)piperidine-1-carboxylate (2e)

Compound **2d** (1.0 g, 2.4 mmol) was dissolved in 2-methyltetrahydrofuran (20 mL), and O-tert-butyl-N,N'-diisopropylisourea (1.21 g, 6 mmol) was added. The mixture was heated to 65 °C. After 3 h of reaction, O-tert-butyl-N,N'-diisopropylisourea (481 mg, 2.4 mmol) was added, and the mixture was stirred overnight. After completion of the reaction as monitored by LCMS, the reaction mixture was filtered. The filtrate was concentrated, and the residue was purified by silica gel flash column chromatography (petroleum ether/ethyl acetate) to give compound **2e** (600 mg, yield 52.8%).

MS (ESI) m/z = 358.7 [M-2C(CH₃)₃+H]⁺.

### Step 5: Preparation of tert-butyl (S)-4-(1-(tert-butoxy)-3-(3-formylphenyl)-1-oxopropan-2-yl)piperidine-1-carboxylate (2f)

Compound **2e** (600 mg, 1.2 mmol), palladium acetate (58 mg, 0.3 mmol), 1,4-bis(diphenylphosphino)butane (102 mg, 0.3 mmol), N-formylsaccharin (622 mg, 3.0 mmol), sodium carbonate (380 mg, 3.6 mmol), and triethylsilane (256 mg, 2.2 mmol) were added to a 20 mL microwave reaction tube. In a nitrogen atmosphere, anhydrous N,N-dimethylformamide (10 mL) was added, and the tube was immediately sealed with a plastic screw cap. The mixture was stirred at room temperature for 10 min, and then the reaction mixture was heated to 75 °C and left to react for 16 h. After completion of the reaction as monitored by LCMS, the reaction mixture was cooled to room temperature, centrifuged, and filtered. The filtrate was purified by reversed-phase flash column chromatography (acetonitrile/water) to give compound **2f** (180 mg, yield 33.7%) as a yellow oil.

MS (ESI): m/z = 440.3 [M+Na]⁺.

### Step 6: Preparation of tert-butyl (S)-4-(3-(3-(aminomethyl)phenyl)-1-(tert-butoxy)-1-oxopropan-2-yl)piperidine-1-carboxylate (2g)

Compound **2f** (80 mg, 0.19 mmol) was dissolved in a 7 M solution of ammonia in methanol (2 mL), and 1 drop of acetic acid was added. After 15 min of stirring at room temperature, sodium cyanoborohydride (150 mg, 2.3 mmol) was added, and the tube was sealed and microwaved at 80 °C for 1 h. After completion of the reaction, the reaction mixture was cooled to room temperature, centrifuged, and filtered. The filtrate was purified by reversed-phase flash column chromatography (acetonitrile/water) to give compound 2g (40 mg, yield 50%) as a yellow oil.

MS (ESI): m/z = 419.4 [M+H]⁺.

### Step 7: Preparation of tert-butyl 4-[(2S)-3-(3-{[bis({3-[(2S)-3-(tert-butoxy)-2-{ 1-[(tert-butoxy)carbonyl]piperidin-4-yl}-3-propionyl]phenyl}methyl)amino]methyl}phenyl)-1-(tert-butoxy)-1-oxopropan-2-yl]piperidine-1-carboxylate (2h)

Compound **2g** (50 mg, 0.14 mmol) was dissolved in absolute methanol (8 mL), and **2f** (120 mg, 0.412 mmol) was added. At room temperature, 1 drop of acetic acid was added, and a molecular sieve was added. After 15 min of stirring, sodium cyanoborohydride (100 mg, 1.4 mmol) was added, and the mixture was heated to 75 °C. After 4 h of reaction, the mixture was left to react overnight at room temperature. After completion of the reaction as monitored by LCMS, a small amount of DMF was added and dissolved. The mixture was centrifuged and filtered. The filtrate was purified by reversed-phase flash column chromatography (acetonitrile/water) to give compound **2h** (100 mg, yield 68.5%) as a white solid.

MS (ESI): m/z = 1221.9 [M+H]⁺.

Step 8: Preparation of (2S)-3-(3-{[bis({3-[(2S)-2-carboxy-2-(piperidin-4-yl)ethyl]phenyl}methyl)amino]methyl}phenyl)-2-(piperidin-4-yl)propanoic acid **(2)** Compound **2h** (100 mg, 0.08 mmol) was dissolved in anhydrous dioxane (0.8 mL), and a 4 M solution of hydrogen chloride in dioxane (2.5 mL) was added. The mixture was left to react at room temperature. After completion of the reaction as monitored by LCMS, the reaction mixture was concentrated, and the crude product was purified by preparative reversed-phase chromatography (acetonitrile/water + 1% formic acid) to give the title compound **2** (30 mg, yield 48.7%).

MS (ESI): m/z = 753.9 [M+H]⁺.

¹H NMR (400 MHz, D₂O) δ 8.46 (s, 1H), 7.45 - 7.32 (m, 6H), 7.27 - 7.18 (m, 6H), 4.30 (s, 6H), 3.55 - 3.38 (m, 6H), 3.06 - 2.91 (m, 9H), 2.77 - 2.67 (m, 3H), 2.43 - 2.32 (m, 3H), 2.23 - 2.13 (m, 3H), 1.96 - 1.76 (m, 6H), 1.63 - 1.45 (m, 6H).

### Example 3

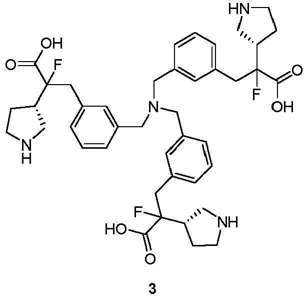

### 3-[3-({Bis[(3-{2-carboxy-2-fluoro-2-[(3S)-pyrrolidin-3-yl]ethyl}phenyl)methyl]amino}methyl)phenyl]-2-fluoro-2-[(3S)-pyrrolidin-3-yl]propanoic acid (3)

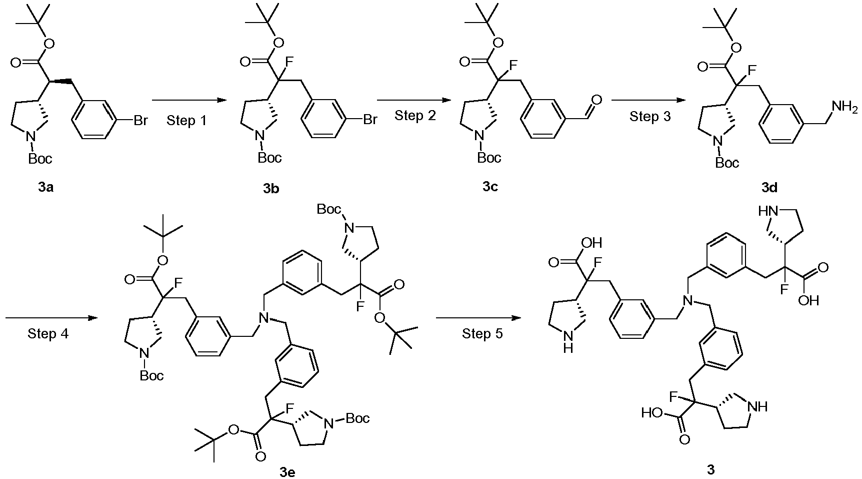

### Step 1: Preparation of tert-butyl (3S)-3-(3-(3-bromophenyl)-1-(tert-butoxy)-2-fluoro-1-oxopropan-2-yl)pyrrolidine-1-carboxylate (3b)

Compound **3a** (2.4 g, 5.28 mmol), prepared using the method in patent WO2020247429, was dissolved in anhydrous tetrahydrofuran (50 mL), and the solution was cooled to - 78 °C. A 2 M solution of lithium diisopropylamide in tetrahydrofuran (5.28 mL) was added. After 30 min of reaction at low temperature, N-fluorobisbenzenesulfonamide (5.0 g, 15.85 mmol) was added. The mixture was gradually warmed to room temperature and left to react for 16 h. After completion of the reaction as monitored by LCMS, saturated ammonium chloride solution was added to the reaction mixture, and extraction was performed with ethyl acetate (20 mL × 2). The organic phases were combined, washed with saturated brine, dried over anhydrous sodium sulfate, and filtered. The filtrate was concentrated to give a crude product, and the crude product was purified by reversed-phase flash column chromatography (acetonitrile/water) to give compound **3b** (2 g, yield 80%).

MS (ESI) m/z = 372.2 [M+H-100]⁺.

### Step 2: Preparation of tert-butyl (3S)-3-(1-(tert-butoxy)-2-fluoro-3-(3-formylphenyl)-1-oxopropan-2-yl)pyrrolidine-1-carboxylate (3e)

Compound **3b** (1000 mg, 2.12 mmol), N-formylsaccharin (894 mg, 4.23 mmol), palladium acetate (48 mg, 0.21 mmol), 1,4-bis(diphenylphosphino)butane (181 mg, 0.42 mmol), and triethylsilane (902 mg, 4.23 mmol) were added to a microwave reaction tube, and N,N-dimethylformamide (15 mL) was then added. After nitrogen purging, the mixture was left to react overnight at 80 °C. After completion of the reaction as monitored by LCMS, saturated ammonium chloride solution was added to the reaction mixture, and extraction was performed with ethyl acetate (20 mL × 2). The organic phases were combined, washed with saturated brine, dried over anhydrous sodium sulfate, and filtered. The filtrate was concentrated to give a crude product, and the crude product was purified by reversed-phase flash column chromatography (acetonitrile/water) to give compound **3c** (350 mg, yield 39%).

MS (ESI) m/z = 444.3 [M+Na]⁺.

### Step 3: Preparation of tert-butyl (3S)-3-(3-(3-(aminomethyl)phenyl)-1-(tert-butoxy)-2-fluoro-1-oxopropan-2-yl)pyrrolidine-1-carboxylate (3d)

Compound **3c** (130 mg, 0.31 mmol) was added to a microwave tube and dissolved with methanol (2 mL), and a 7 M solution of ammonia in methanol (2 mL) and 1 drop of acetic acid were added. After 30 min of stirring at room temperature, sodium cyanoborohydride (92 mg, 1.54 mmol) was added, and the mixture was microwaved at 80 °C for 1 h. After completion of the reaction as monitored by LCMS, the reaction mixture was concentrated under reduced pressure to give a crude product, and the crude product was purified by reversed-phase flash column chromatography (acetonitrile/water) to give compound **3d** (50 mg, yield 38%) as a pale yellow oil.

MS (ESI) m/z = 423.6 [M+H]⁺.

### Step 4: Preparation of tert-butyl (3S)-3-[3-(3-{[bis({3-[3-(tert-butoxy)-2-[(3S)-1-[(tert-butoxy)carbonyl]pyrrolidin-3-yl]-2-fluoro-3-propionyl]phenyl}methyl)amino]methyl}phenyl)-1-(tert-butoxy)-2-fluoro-1-oxopropan-2-yl]pyrrolidine-1-carboxylate (3e)

Compound **3d** (50 mg, 0.12 mmol) and **3c** (100 mg, 0.24 mmol) were dissolved in methanol (20 mL), and 1 drop of acetic acid was added. After 30 min of stirring at room temperature, sodium cyanoborohydride (92 mg, 1.54 mmol) was added. The mixture was left to react at 60 °C for 5 h. After completion of the reaction as monitored by LCMS, the reaction mixture was concentrated under reduced pressure to give a crude product, and the crude product was purified by reversed-phase flash column chromatography (acetonitrile/water) to give compound **3e** (80 mg, yield 55%) as a pale yellow oil.

MS (ESI) m/z = 1233.9 [M+H]⁺.

### Step 5: Preparation of 3-[3-({bis[(3-{2-carboxy-2-fluoro-2-[(3S)-pyrrolidin-3-yl]ethyl}phenyl)methyl]amino}methyl)phenyl]-2-fluoro-2-[(3S)-pyrrolidin-3-yl]propanoic acid (3)

Compound **3e** (80 mg, 0.07 mmol) was dissolved in dioxane (2 mL), and a 4 M solution of hydrogen chloride in dioxane (2 mL) was added. The mixture was left to react at room temperature for 2 h. After completion of the reaction, the reaction mixture was concentrated under reduced pressure to give a crude product, and the crude product was purified by preparative reversed-phase chromatography (acetonitrile/water + 1% formic acid) to give the title compound **3** (20 mg, yield 40%).

MS (ESI) m/z = 765.6 [M+H]⁺.

¹H NMR (400 MHz, D₂O) δ 8.45 (s, 1.5H), 7.41 (t, *J* = 7.5 Hz, 3H), 7.34 (d, *J* = 7.7 Hz, 3H), 7.32 - 7.25 (m, 6H), 4.17 (s, 6H), 3.59 - 3.44 (m, 3H), 3.43 - 3.25 (m, 6H), 3.22 (s, 3H), 3.20 - 3.09 (m, 6H), 3.09 - 2.95 (m, 3H), 2.40 - 2.27 (m, 2H), 2.19 - 2.06 (m, 3H), 2.02 - 1.90 (m, 1H).

### Example 4

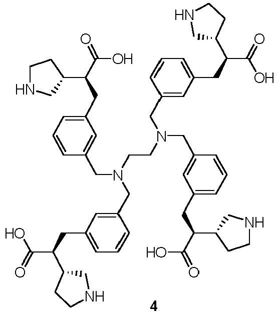

### (2S)-3-{3-[({2-[Bis({3-[(2S)-2-carboxy-2-[(3R)-pyrrolidin-3-yl]ethyl]phenyl}methyl)amino]ethyl}({3-[(2S)-2-carboxy-2-[(3R)-pyrrolidin-3-yl]ethyl]phenyl}methyl)amino)methyl]phenyl}-2-[(3R)-pyrrolidin-3-yl]propanoic acid

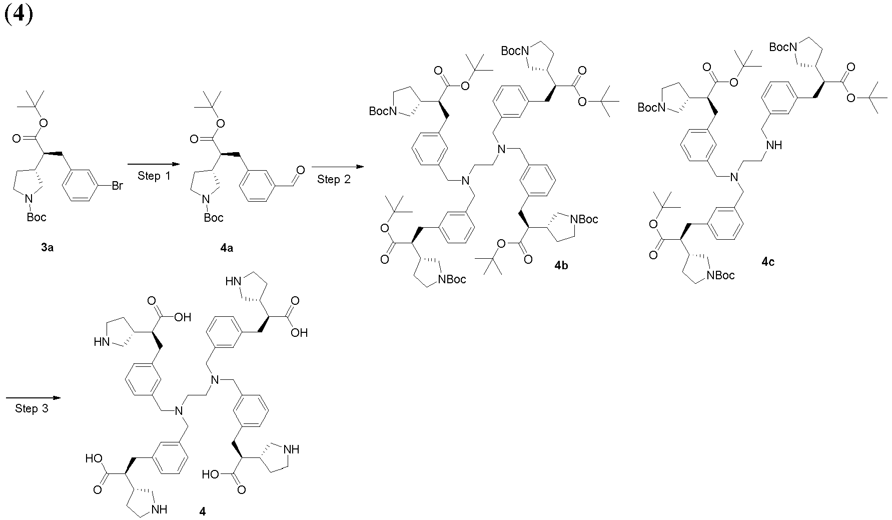

### Step 1: Preparation of tert-butyl (R)-3-((S)-1-(tert-butoxy)-3-(3-formylphenyl)-1-oxopropan-2-yl)pyrrolidine-1-carboxylate (4a)

Compound **3a** (400 mg, 0.9 mmol), N-formylsaccharin (428 mg, 2.0 mmol), palladium acetate (40 mg, 0.2 mmol), 1,4-bis(diphenylphosphino)butane (83 mg, 0.2 mmol), and triethylsilane (0.24 mL, 1.5 mmol) were added to a microwave reaction tube, and N,N-dimethylformamide (6 mL) was then added. After nitrogen purging, the mixture was left to react overnight at 75 °C. After completion of the reaction as monitored by LCMS, the reaction mixture was cooled to room temperature, centrifuged, and filtered. The filtrate was purified by reversed-phase flash column chromatography (acetonitrile/water) to give compound **4a** (270 mg, yield 76%) as a yellow oil.

MS (ESI): m/z = 404.5 [M+H]⁺.

### Step 2: Preparation of tert-butyl (3R)-3-[(2S)-3-{3-[({2-[bis({3-[(2S)-3-(tert-butoxy)-2-[(3R)-1-[(tert-butoxy)carbonyl]pyrrolidin-3-yl]-3-propionyl]phenyl}methyl)amino]ethyl}({3-[(2S)-3-(tert-butoxy)-2-[(3R)-1-[(tert-butoxy)carbonyl]pyrrolidin-3-yl]-3-propionyl]phenyl}methyl)amino)methyl]phenyl}-1-(tert-butoxy)-1-oxopropan-2-yl]pyrrolidine-1-carboxylate (4b) and tert-butyl (3R)-3-[(2S)-1-(tert-butoxy)-3-(3-{[({3-[(2S)-3-(tert-butoxy)-2-[(3R)-1-[(tert-butoxy)carbonyl]pyrrolidin-3-yl]-3-propionyl]phenyl}methyl)({2-[({3-[(2S)-3-(tert-butoxy)-2-[(3R)-1-[(tert-butoxy)carbonyl]pyrrolidin-3-yl]-3-propionyl] phenyl }methyl)amino]ethyl })amino]methyl } phenyl)-1 -oxopropan-2-yl]pyrrolidine-1-carboxylate (4c)

Ethylenediamine (11 mg, 0.18 mmol) and compound **4a** (320 mg, 0.79 mmol) were dissolved in methanol (20 mL), and 1 drop of acetic acid was added. After 30 min of stirring at room temperature, sodium cyanoborohydride (60 mg, 0.99 mmol) was added, and the mixture was heated to 60 °C and left to react for 5 h. After completion of the reaction as monitored by LCMS, the reaction mixture was concentrated under reduced pressure to give a crude product, and the crude product was purified by reversed-phase flash column chromatography (water/acetonitrile) to give compound **4b** (50 mg, yield 16%) and compound **4c** (150 mg, yield 62%) as pale yellow oils.

Compound **4b:** MS (ESI) m/z = 1610.2 [M+H]⁺.

Compound **4c:** MS (ESI) m/z = 1222.6 [M+H]⁺.

### Step 3: Preparation of (2S)-3-{3-[({2-[bis({3-[(2S)-2-carboxy-2-[(3R)-pyrrolidin-3-yl]ethyl]phenyl}methyl)amino]ethyl}({3-[(2S)-2-carboxy-2-[(3R)-pyrrolidin-3-yl]ethyl]phenyl}methyl)amino)methyl]phenyl}-2-[(3R)-pyrrolidin-3-yl]propanoic acid (4)

Compound **4b** (50 mg, 0.07 mmol) was dissolved in dioxane (2 mL), and a 4 M solution of hydrogen chloride in dioxane (2 mL) was added. The mixture was left to react at room temperature for 2 h. After completion of the reaction as monitored by LCMS, the reaction mixture was concentrated under reduced pressure to give a crude product, and the crude product was purified by preparative reversed-phase chromatography (acetonitrile/water + 1% formic acid) to give the title compound **4** (10 mg, yield 87%).

MS (ESI) m/z = 985.3 [M+H]⁺.

¹H NMR (400 MHz, D₂O) δ 8.44 (s, 3H), 7.39 - 7.32 (m, 4H), 7.31 - 7.26 (m, 4H), 7.18 - 7.06 (m, 8H), 3.88 (s, 8H), 3.55 - 3.47 (m, 4H), 3.44 - 3.36 (m, 4H), 3.31 - 3.17 (m, 4H), 3.05 - 2.87 (m, 8H), 2.84 - 2.71 (m, 8H), 2.55 - 2.37 (m, 8H), 2.21 - 2.03 (m, 4H), 1.84 - 1.62 (m, 4H).

### Example 5

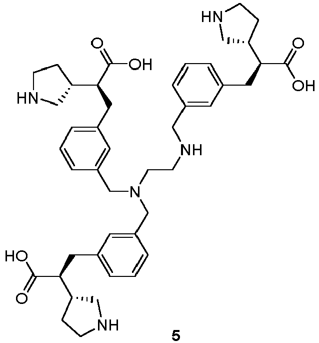

### (2S)-3-(3-{[({3-[(2S)-2-Carboxy-2-[(3R)-pyrrolidin-3-yl]ethyl]phenyl}methyl)({2-[({3-[(2S)-2-carboxy-2-[(3R)-pyrrolidin-3-yl]ethyl]phenyl}methyl)amino]ethyl})amino]methyl}phenyl)-2-[(3R)-pyrrolidin-3-yl]propanoic acid (5)

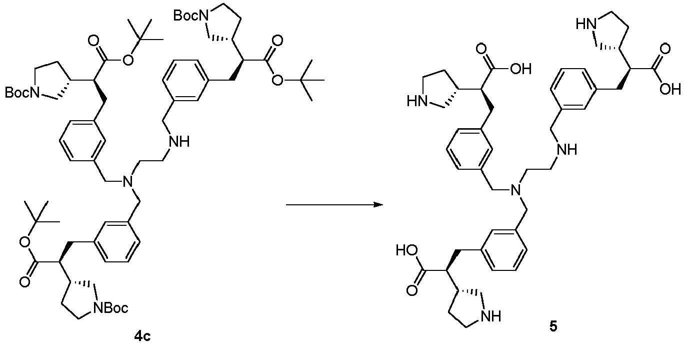

Compound **4**c (80 mg, 0.06 mmol) was dissolved in dioxane (2 mL), and a 4 M solution of hydrogen chloride in dioxane (2 mL) was added. The mixture was left to react at room temperature for 2 h. After completion of the reaction as monitored by LCMS, the reaction mixture was concentrated under reduced pressure to give a crude product, and the crude product was purified by preparative reversed-phase chromatography (acetonitrile/water + 1% formic acid) to give the title compound 5 (20 mg, yield 40%).

MS (ESI) m/z = 754.5 [M+H]⁺.

¹H NMR (400 MHz, D₂O) δ 8.43 (s, 3H), 7.44 - 7.31 (m, 6H), 7.30 - 7.24 (m, 4H), 7.23 - 7.17 (m, 2H), 4.28 - 4.03 (m, 6H), 3.66 - 3.53 (m, 3H), 3.48 - 3.39 (m, 3H), 3.33 - 2.97 (m, 10H), 2.91 - 2.73 (m, 6H), 2.57 - 2.40 (m, 6H), 2.24 - 2.05 (m, 3H), 1.83 - 1.66 (m, 3H).

### Example 6

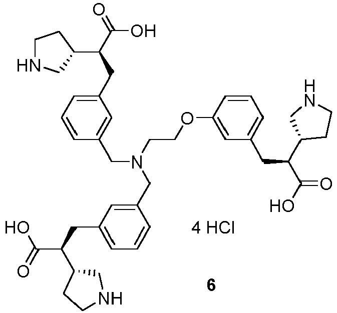

### (2S)-3-(3-{[(2-{3-[(2S)-2-Carboxy-2-[(3R)-pyrrolidin-3-yl]ethyl]phenoxy}ethyl)({3-[(2S)-2-carboxy-2-[(3R)-pyrrolidin-3-yl]ethyl]phenyl}methyl)amino]methyl}phenyl)-2-[(3R)-pyrrolidin-3-yl]propanoic acid tetrahydrochloride (6)

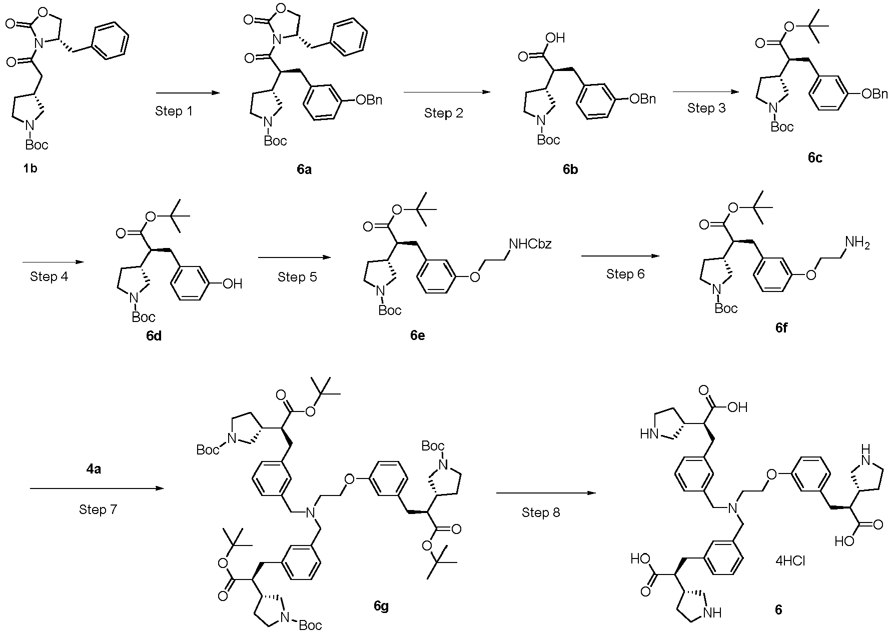

### Step 1: Preparation of tert-butyl (R)-3-((S)-1-((S)-4-benzyl-2-oxooxazolidin-3-yl)-3-(3-(benzyloxy)phenyl)-1-oxopropan-2-yl)pyrrolidine-1-carboxylate (6a)

Compound **1b** (10.0 g, 25.74 mmol) was dissolved in tetrahydrofuran (10 mL) in a nitrogen atmosphere, and the solution was cooled to -30 °C. A 1 M solution of lithium bis(trimethylsilyl)amide in tetrahydrofuran (31 mL, 31 mmol) was added dropwise. After the addition, the mixture was left to react for 30 min. A solution of 3-benzyloxybromobenzyl (8.56 g, 30.89 mmol) in tetrahydrofuran (15 mL) was added dropwise. After 1 h of reaction, the mixture was warmed to 0 °C-5 °C and left to react overnight. Saturated ammonium chloride solution (50 mL) was added, and extraction was performed with methyl tert-butyl ether (50 mL × 2). The organic phase was washed with saturated sodium chloride solution (70 mL), dried over anhydrous sodium sulfate, and filtered. The filtrate was concentrated under reduced pressure, and the resulting residue was purified by column chromatography (petroleum ether:ethyl acetate = 4:1) to give compound **6a** (7.08 g, yield: 47.0%).

### Step 2: Preparation of (S)-3-(3-(benzyloxy)phenyl)-2-((R)-1-(tert-butoxycarbonyl)pyrrolidin-3-yl)propanoic acid (6b)

Compound **6a** (7 g, 11.983 mmol) was dissolved in tetrahydrofuran (70 mL) and water (35 mL), and hydrogen peroxide (30%) (4.07 g, 35.95 mmol) and lithium hydroxide monohydrate (431 mg, 17.97 mmol) were added. The mixture was left to react overnight at room temperature. The pH was adjusted to >13 with a 2 M sodium hydroxide solution, and extraction was performed with methyl tert-butyl ether (50 mL × 2). The pH of the aqueous phase was adjusted to <3 with a 2 M hydrochloric acid solution, and extraction was performed with methyl tert-butyl ether (50 mL × 2). The organic phase was washed with saturated sodium chloride solution (70 mL), dried over anhydrous sodium sulfate, and filtered. The filtrate was concentrated to dryness under reduced pressure to give a crude product of compound **6b** (3.11 g). The product was directly used in the next step without purification.

### Step 3: Preparation of tert-butyl (R)-3-((S)-3-(3-(benzyloxy)phenyl)-1-(tert-butoxy)-1-oxopropan-2-yl)pyrrolidine-1-carboxylate (6e)

The crude product of compound **6b** (3.1 g, 7.29 mmol) from the previous step was dissolved in 2-methyltetrahydrofuran (60 mL), and 2-tert-butyl-1,3-diisopropylisourea (5.84 g, 29.14 mmol) was added. The mixture was heated to 65 °C and left to react overnight. The reaction mixture was filtered, and the filtrate was concentrated to dryness under reduced pressure. The resulting residue was purified by column chromatography (petroleum ether:ethyl acetate = 5:1) to give compound 6c (2.5 g, yield over two steps: 43.3%).

### Step 4: Preparation of tert-butyl (R)-3-((S)-1-(tert-butoxy)-3-(3-hydroxyphenyl)-1-oxopropan-2-yl)pyrrolidine-1-carboxylate (6d)

Compound **6c** (2.5 g, 5.19 mmol) was dissolved in MeOH (40 mL) in a hydrogen atmosphere, and Pd/C (0.80 g) was added. The mixture was left to react overnight at room temperature. The reaction mixture was filtered, and the filtrate was concentrated under reduced pressure to give compound **6d** (1.78 g, yield: 87.6%).

Step 5: Preparation of tert-butyl (R)-3-((S)-3-(3-(2-(((benzyloxy)carbonyl)amino) ethoxy)phenyl)-1-(tert-butoxy)-1-oxopropan-2-yl)pyrrolidine-1-carboxylate **(6e)** Compound **6d** (400 mg, 0.99 mmol), benzyl N-(2-bromoethyl)carbamate (280 mg, 1.09 mmol), and cesium carbonate (803 mg, 2.47 mmol) were added to N,N-dimethylformamide (8 mL), and the mixture was heated to 80 °C and left to react for 3 h. Water (40 mL) was added, and extraction was performed with ethyl acetate (30 mL × 2). The organic phase was washed with saturated sodium chloride solution (50 mL), dried over anhydrous sodium sulfate, and filtered. The filtrate was concentrated under pressure to give a residue, and the residue was purified by column chromatography (petroleum ether:ethyl acetate = 8:1) to give compound **6e** (300 mg, yield 53.5%).

### Step 6: Preparation of tert-butyl (R)-3-((S)-3-(3-(2-aminoethoxy)phenyl)-1-(tert-butoxy)-1-oxopropan-2-yl)pyrrolidine-1-carboxylate (6f)

Compound **6e** (300 mg, 0.528 mmol) was dissolved in MeOH (5 mL) in a hydrogen atmosphere, and Pd/C (100 mg) was added. The mixture was left to react overnight at room temperature. The reaction mixture was filtered, and the filtrate was concentrated under reduced pressure to give compound **6f (150** mg, yield 65.4%).

### Step 7: Preparation of tert-butyl (3R)-3-[(2S)-1-(tert-butoxy)-3-(3-{[(2-{3-[(2S)-3-(tert-butoxy)-2-[(3R)-1-[(tert-butoxy)carbonyl]pyrrolidin-3-yl]-3-oxopropyl]phenoxy}ethyl)({3-[(2S)-3-(tert-butoxy)-2-[(3R)-1-[(tert-butoxy)carbonyl]pyrrolidin-3-yl]-3-oxopropyl]phenyl}methyl)amino]methyl}phenyl)-1-oxopropan-2-yl]pyrrolidine-1-carboxylate (6g)

Compound **6f** (150 mg, 0.35 mmol) and compound **4a** (278.6 mg, 0.69 mmol) were dissolved in isopropanol (6 mL) under an ice bath, and 1 drop of glacial acetic acid was added. The mixture was incubated for 30 min. Sodium triacetoxyborohydride (292.6 mg, 1.38 mmol) was added, and the mixture was slowly warmed to room temperature and left to react overnight. The pH was adjusted to 8-9 with saturated sodium bicarbonate solution, and extraction was performed with ethyl acetate (40 mL × 2). The organic phase was washed with saturated sodium chloride solution (60 mL), dried over anhydrous sodium sulfate, and filtered. The filtrate was concentrated to give a crude product (480 mg), and the crude product was purified by preparative liquid chromatography to give compound **6g** (250 mg, yield 59.9%).

### Step 8: Preparation of (2S)-3-(3-{[(2-{3-[(2S)-2-carboxy-2-[(3R)-pyrrolidin-3-yl]ethyl]phenoxy}ethyl)({3-[(2S)-2-carboxy-2-[(3R)-pyrrolidin-3-yl]ethyl]phenyl}methyl)amino]methyl}phenyl)-2-[(3R)-pyrrolidin-3-yl]propanoic acid tetrahydrochloride (6)

Compound **6g** (100 mg, 0.083 mmol) was added to a 4 M solution of hydrochloric acid in dioxane (4 mL), and the mixture was stirred overnight at room temperature. The supernatant was removed to give a solid, and the solid was washed with ethyl acetate and lyophilized to give compound 6 (50 mg, yield 68.5%).

MS m/z (ESI): 741.3 [M+H]⁺.

¹H NMR (400 MHz, D₂O): *δ* 7.42-7.16 (m, 9H), 6.86 (d, *J* = 7.6 Hz, 1H), 6.70 (dd, *J* = 8.3, 2.6 Hz, 1H), 6.65 (s, 1H), 4.43 (s, 4H), 4.14-3.97 (m, 2H), 3.62-3.45 (m, 5H), 3.45-3.30 (m, 3H), 3.29-3.14 (m, 3H), 3.07-2.98 (m, 3H), 2.93-2.63 (m, 9H), 2.61-2.39 (m, 3H), 2.24-2.02 (m, 3H), 1.78-1.64 (m, 3H).

### Example 7

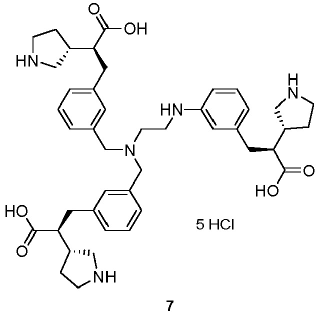

### (2S)-3-[3-({[2-({3-[(2S)-2-Carboxy-2-[(3R)-pyrrolidin-3-yl]ethyl]phenyl}amino)ethyl]({3-[(2S)-2-carboxy-2-[(3R)-pyrrolidin-3-yl]ethyl]phenyl}methyl)amino}methyl)phenyl]-2-[(3R)-pyrrolidin-3-yl]propanoic acid pentahydrochloride (7)

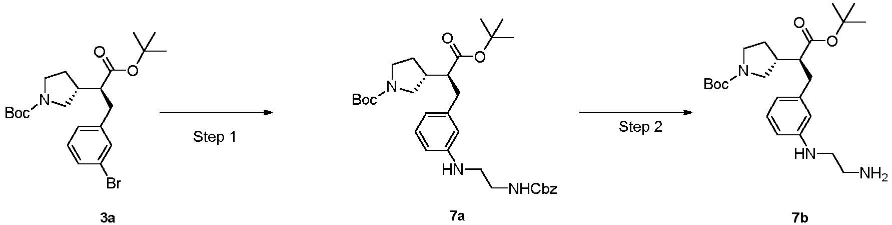

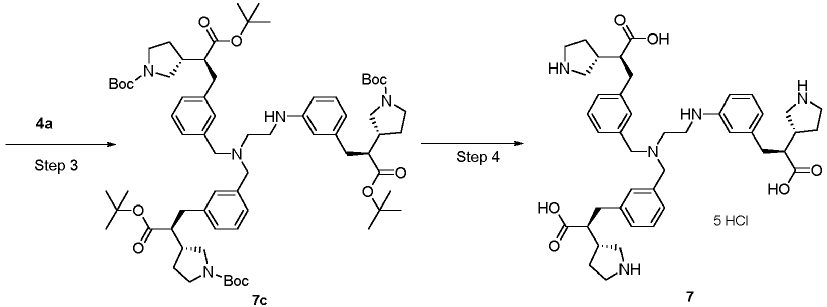

Step 1: Preparation of tert-butyl (R)-3-((S)-3-(3-((2-(((benzyloxy)carbonyl)amino) ethyl)amino)phenyl)-1-(tert-butoxy)-1 -oxopropan-2-yl)pyrrolidine-1 -carboxylate **(7a)** Compound **3a** (700 mg, 1.54 mmol), N-benzyloxycarbonylethylenediamine (448.81 mg, 2.31 mmol), BrettPhos Pd G3 (49.9 mg, 0.06 mmol), potassium carbonate (638.7 mg, 4.62 mmol), and dioxane (10 mL) were weighed into a reaction flask, and the system was purged with nitrogen gas three times. The mixture was left to react overnight at 100 °C. LCMS monitoring showed there was a product generated. Ethyl acetate and water were added to the reaction mixture, and the mixture was separated. The organic phase was washed with saturated brine, dried over anhydrous sodium sulfate, and filtered. The filtrate was concentrated, and the residue was purified by column chromatography to give compound 7a (400 mg, 45.7%).

### Step 2: Preparation of tert-butyl (R)-3-((S)-3-(3-((2-aminoethyl)amino)phenyl)-1-(tert-butoxy)-1-oxopropan-2-yl)pyrrolidine-1-carboxylate (7b)

Compound 7a (400 mg, 0.71 mmol) and 10% Pd/C (100 mg) were weighed into a reaction flask, and methanol (10 mL) was added. The system was purged with nitrogen gas three times and with hydrogen gas three times, and the mixture was left to react overnight under a hydrogen balloon. LCMS monitoring showed there was a product generated. The reaction mixture was filtered, and the filtrate was concentrated to give a crude product of compound **7b** (200 mg, 65.5%).

### Step 3: Preparation of tert-butyl (3R)-3-[(2S)-1-(tert-butoxy)-3-[3-({[2-({3-[(2S)-3-(tert-butoxy)-2-[(3R)-1-[(tert-butoxy)carbonyl]pyrrolidin-3-yl]-3-oxopropyl]phenyl}amino)ethyl]({3-[(2S)-3-(tert-butoxy)-2-[(3R)-1-[(tert-butoxy)carbonyl]pyrrolidin-3-yl]-3-oxopropyl]phenyl}methyl)amino}methyl)phenyl]-1-oxopropan-2-yl]pyrrolidine-1-carboxylate (7c)

Compounds **7b** (150 mg, 0.35 mmol) and **4a** (349 mg, 0.87 mmol) were weighed into a reaction flask. Isopropanol (10 mL) was added, and one drop of acetic acid was added. The mixture was stirred for 1 h under an ice bath, and sodium triacetoxyborohydride (218.9 mg, 1.04 mmol) was then added. The mixture was left to react overnight. Saturated sodium bicarbonate solution and ethyl acetate were added to the reaction mixture, and the mixture was separated. The organic phase was washed with saturated brine, dried over anhydrous sodium sulfate, and filtered. The filtrate was concentrated to give a crude product, and the crude product was purified by preparative liquid chromatography to give compound **7c** (100 mg, 23.9%).

### Step 4: Preparation of (2S)-3-[3-({[2-({3-[(2S)-2-carboxy-2-[(3R)-pyrrolidin-3-yl]ethyl]phenyl}amino)ethyl]({3-[(2S)-2-carboxy-2-[(3R)-pyrrolidin-3-yl]ethyl]phenyl}methyl)amino}methyl)phenyl]-2-[(3R)-pyrrolidin-3-yl]propanoic acid pentahydrochloride (7)

Compound **7c** (100 mg, 8.3 mmol) was weighed out, and a 4 M solution of hydrochloric acid in dioxane (10 mL) was added at room temperature. The mixture was stirred overnight. The supernatant was removed to give a solid. The solid was washed with ethyl acetate and dissolved in water, and the solution was lyophilized to give compound 7 (70 mg, 72.1%).

MS m/z (ESI): 740.4 [M+1]⁺.

¹H NMR (400 MHz, D₂O): *δ* 7.45-7.21 (m, 8H), 7.12 (t, *J =* 7.8 Hz, 1H), 6.75 (d, *J =* 7.5 Hz, 1H), 6.53-6.38 (m, 2H), 4.41 (s, 4H), 3.68-3.51 (m, 2H), 3.51-3.39 (m, 3H), 3.38-3.18 (m, 8H), 3.15-2.99 (m, 2H), 2.98-2.67 (m, 9H), 2.66-2.46 (m, 4H), 2.26-2.06 (m, 3H), 1.84-1.69 (m, 3H).

### Biological Assays

### Test Example 1: Test for Activity of Compounds in Inhibiting Lp(a) Assembly

The disclosure used a double antibody ELISA method to measure the efficiency of Apo(a) and ApoB protein assembly. The antibodies were ApoB-Capture antibody (Mabtech) and Apo(a)-Detector antibody (Abcam); the test samples were plasma from human transgenic hApo(a) and hApoB mice and were diluted 500-fold before use.

Experimental steps: Equal amounts of Apo(a) and ApoB serum were mixed with a serially diluted test compound (highest concentration at 100 nM, serially diluted 3-fold). The mixture was incubated in a 37 °C incubator for 2 h. The reaction was then terminated by adding 6-aminocaproic acid (EACA, purchased from Sigma) to a final concentration of 150 mM. The post-reaction solution was then added to an ELISA plate pre-coated with ApoB-Capture antibody, and the plate was incubated at room temperature for 2 h. Then the plate was washed 4 times with wash buffer; biotinylated Apo(a)-Detector antibody was added, and the plate was incubated at room temperature for 1 h. The plate was washed, and the chromogenic substrate 3,3',5,5'-tetramethylbenzidine (TMB, purchased from Abcam) solution was added. After the plate was incubated at room temperature for 15 min, a reaction stop solution was added. Immediately after the solution was mixed well, the absorbance at 450 nm was measured using a microplate reader. Finally, data analysis and IC₅₀ calculation were performed using GraphPad Prism 9 software.

The 0% inhibition rate for Apo(a) and ApoB protein assembly corresponds to the OD value where the compound concentration was 0 (1% DMSO); the 100% inhibition rate for Apo(a) and ApoB protein assembly corresponds to the OD value where only ApoB protein solution (a dilution of plasma from hApoB mice) was added.

### Experimental results:

**Table 1. The activity of the compounds of the disclosure in inhibiting Lp(a) assembly**

| Compound | Lp(a) assembly, IC₅₀ (nM) |
|---|---|
| **1** | 0.75 |
| **2** | 1.7 |
| **3** | 1.4 |
| **4** | 0.85 |
| **5** | 0.78 |
| **6** | 0.41 |
| **7** | 0.76 |

Conclusion: The compounds of the disclosure exhibited significant inhibitory effects on Lp(a) assembly.

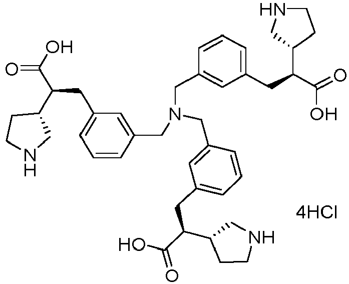

### (Reference 1, prepared by reference to the method of Example 1 of CN114008021)

### Test Example 2: In Vivo Pharmacokinetic Experiment on Beagles

Beagles were used as test animals. After intragastric administration of the compound of the disclosure to beagles, plasma concentrations at different time points were determined by LC/MS/MS. The pharmacokinetic behavior of the compound of the disclosure in beagles was studied and its pharmacokinetic profile was evaluated.

### Test animals: 2 healthy male beagles aged 8-36 months per group

Compound solution preparation: A certain amount of compound was weighed out and normal saline was added to form a 2 mg/mL colorless clear solution.

Administration: Beagles were fasted overnight and then intragastrically dosed. Reference 1 and compound 6 were both administered at a dose of 10 mg/kg.

Procedure: Beagles were intragastrically administered the compound of the disclosure. Blood samples of about 0.6 mL were collected from the peripheral vein at 0.25, 0.5, 1, 2, 4, 6, 8, and 24 h post-dose and placed into test tubes containing EDTA-K2. The blood samples were centrifuged at about 4 °C at 2000 rpm for 10 min to separate plasma, and the plasma was stored at -75 °C.

Determination of the content of the test compound in beagle plasma at these different blood collection time points: Samples of 33 µL (30 µL of plasma and 3 µL of blank) of the beagle plasma collected at these time points post-dose were taken and added to 18 µL of a 6% perchloric acid solution. After 30 s of vortexing, the mixture was centrifuged at a temperature of 4 °C at 3900 rpm for 15 min, and 200 µL of alkalinized (the pH was adjusted to 10-11 with ammonia water) acetonitrile solution containing dexamethasone as an internal standard was then added to precipitate protein. After 30 s of vortexing, the mixture was centrifuged at 4 °C at 3900 rpm for 15 min. The supernatants of the plasma samples were taken and 3-fold diluted with water, and 8 µL of dilution was taken for LC/MS/MS analysis.

### Pharmacokinetic parameters

After plasma concentrations were determined by LC/MS/MS analysis, pharmacokinetic parameters were calculated using WinNonlin 6.1 software and a non-compartmental model method. Pharmacokinetic parameters of the compound of the disclosure in beagles are shown in Table 2 below.

**Table 2. Pharmacokinetic parameters of the compound of the disclosure after oral administration to beagles**

| No. | Dose | Maximum plasma concentration | Area under curve | Half-life |
|---|---|---|---|---|
| | mg/kg | Cmax (ng/mL) | AUC (ng/mL*h) | T_{1/2} (h) |
| Reference 1 | 10 | 1628 | 22950 | 20.7 |
| Compound 6 | 10 | 2473 | 28594 | 18.3 |

Conclusion: Compound 6 exhibited better oral absorption in beagles, resulting in better plasma exposure and a longer half-life. It has an excellent pharmacokinetic profile and, when orally administered, has significant advantages.

### Test Example 3: In Vivo Pharmacokinetic Experiment on Cynomolgus Monkeys

Cynomolgus monkeys were used as test animals. After intragastric administration of the compound of the disclosure to cynomolgus monkeys, plasma concentrations at different time points were determined by LC/MS/MS. The pharmacokinetic behavior of the compound of the disclosure in cynomolgus monkeys was studied and its pharmacokinetic profile was evaluated.

### Test animals: 2 healthy cynomolgus monkeys aged 24-36 months per group (one male and one female)

Compound solution preparation: Certain amounts of compound were weighed out and normal saline was added to form 10 mg/mL (reference 1) and 3 mg/mL (compound 6) colorless clear solutions.

Administration: Cynomolgus monkeys were fasted overnight and then intragastrically dosed. Reference 1 was administered at a dose of 50 mg/kg, and compound 6 was administered at a dose of 15 mg/kg.

Procedure: Cynomolgus monkeys were intragastrically administered the compound of the disclosure. Blood samples of about 0.5 mL were collected from the peripheral vein at 0.25, 0.5, 1, 2, 4, 6, 8, and 24 h post-dose and placed into test tubes containing EDTA-K2. The blood samples were centrifuged at about 4 °C at 2000 rpm for 10 min to separate plasma, and the plasma was stored at -75 °C.

Determination of the content of the test compound in cynomolgus monkey plasma at these different blood collection time points: Samples of 33 µL (30 µL of plasma and 3 µL of blank) of the cynomolgus monkey plasma collected at these time points post-dose were taken and added to 18 µL of a 6% perchloric acid solution. After 30 s of vortexing, the mixture was centrifuged at a temperature of 4 °C at 3900 rpm for 15 min, and 200 µL of alkalinized (the pH was adjusted to 10-11 with ammonia water) acetonitrile solution containing dexamethasone as an internal standard was then added to precipitate protein. After 30 s of vortexing, the mixture was centrifuged at 4 °C at 3900 rpm for 15 min. The supernatants of the plasma samples were taken and 3-fold diluted with water, and 8 µL of dilution was taken for LC/MS/MS analysis.

### Pharmacokinetic parameters

After plasma concentrations were determined by LC/MS/MS analysis, pharmacokinetic parameters were calculated using WinNonlin 6.1 software and a non-compartmental model method. Pharmacokinetic parameters of the compound of the disclosure in cynomolgus monkeys are shown in Table 3 below.

**Table 3. Pharmacokinetic parameters of the compound of the disclosure after oral administration to cynomolgus monkeys**

| No. | Dose | Maximum plasma concentration | Area under curve | Unit-dose area under curve | Half-life |
|---|---|---|---|---|---|
| | mg/kg | Cmax (ng/mL) | AUC (ng/mL*h) | AUC/D (mg/mL*h) | T_{1/2} (h) |
| Reference 1 | 50 | 2192 | 22153 | 443 | 17.7 |
| Compound 6 | 15 | 2367 | 33067 | 2204 | 16.6 |

Conclusion: Compound 6 exhibited better oral absorption in cynomolgus monkeys, and its plasma exposure was nearly 5 times that of reference 1, indicating that the pharmacokinetic properties of the compound of the invention are significantly better than those of reference 1.

### Test Example 4: High-Dose Single-Dose Tolerance Experiment on Mice

C57/6J mice were used as test animals and administered a high dose of the compound of the disclosure by oral gavage. Clinical observations were carried out on the mice, and the safety of the compound of the disclosure was evaluated.

Experimental method: 8 male C57/6J mice were numbered 1-8 and weighed. The mice were fasted for 4 h before the experiment and weighed after the fast (0 h). Six mice with body weights closer to the average were selected and divided into 2 groups of 3.

The first group (G1) was administered reference 1, and the second group (G2) was administered the compound of the disclosure, Example 6. In both groups G1 and G2, the compounds were administrated by oral gavage at a dose of 1000 mg/kg, with normal saline as a vehicle. Clinical observations were carried out on the mice in the experimental group at 15 min, 30 min, 1 h, 2 h, 4 h, 6 h, and 24 h post-dose, and both groups were given food at 2 h post-dose. The body weight of the mice was monitored for 7 consecutive days, and the results are shown in Table 4 below.

**Table 4. The high-dose single-dose tolerance experiment of the compound of the disclosure on mice**

| Group | Mouse body weight (g)^{a} | | | |
|---|---|---|---|---|
| | Day 0 | Day 3 | Day 5 | Day 7 |
| Reference 1 | 25.87 | 25.70 | 25.10 | 24.53 |
| Compound 6 | 25.83 | 26.93 | 26.70 | 26.67 |

| | | | | |
|---|---|---|---|---|
| Note: a. means | | | | |

Conclusion: 7 d after the high-dose (1000 mg/kg) administration, there was a significant downward trend in the body weight of the mice in the reference 1 group, and the body weight of the mice in the corresponding compound 6 group remained unchanged or even increased. The compound of the disclosure is expected to be capable of demonstrating superior safety over a longer period of administration.

## Claims

1. A compound represented by formula II or a pharmaceutically acceptable salt thereof, 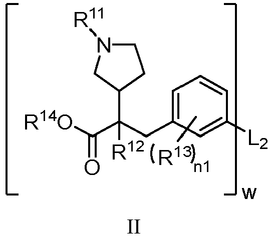
wherein R¹¹ is independently selected from the group consisting of hydrogen or C₁₋₆ alkyl, wherein the alkyl is optionally substituted with one or more R^{11A}, and each R^{11A} is independently selected from the group consisting of halogen, hydroxy, cyano, or amino; R¹² is independently selected from the group consisting of hydrogen, deuterium, halogen, or C₁₋₆ alkyl, wherein the alkyl is optionally substituted with one or more R^{12A}, and each R^{12A} is independently selected from the group consisting of halogen, hydroxy, cyano, or amino;
R¹³ is independently selected from the group consisting of hydrogen, deuterium, halogen, cyano, C₁₋₆ alkyl, or C₁₋₆ alkoxy, wherein the alkyl or alkoxy is optionally substituted with one or more R^{13A}, and each R^{13A} is independently selected from the group consisting of halogen, hydroxy, cyano, or amino;
R¹⁴ is independently selected from the group consisting of hydrogen or C₁₋₆ alkyl;
W is selected from the group consisting of 3 or 4;
L₂ is selected from the group consisting of 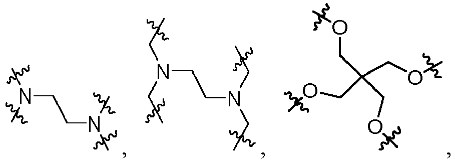 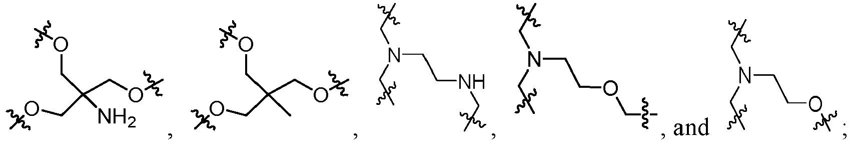
n1 is selected from the group consisting of integers between 0-4.

2. The compound or the pharmaceutically acceptable salt thereof according to claim 1, wherein R¹² is independently selected from the group consisting of hydrogen or deuterium.

3. The compound or the pharmaceutically acceptable salt thereof according to claim 1 or 2, wherein R¹² is independently selected from halogen, e.g., fluorine or chlorine; or R¹² is independently selected from C₁₋₆ alkyl, wherein the alkyl is optionally substituted with one or more R^{12A}, and R^{12A} is as defined in claim 1.

4. The compound or the pharmaceutically acceptable salt thereof according to any of claims 1-3, wherein R¹¹ is independently selected from hydrogen.

5. The compound or the pharmaceutically acceptable salt thereof according to any of claims 1-4, wherein R¹³ is independently selected from the group consisting of hydrogen or deuterium.

6. The compound or the pharmaceutically acceptable salt thereof according to any of claims 1-4, wherein R¹³ is independently selected from the group consisting of deuterium or C₁₋₆ alkoxy, wherein the alkoxy is optionally substituted with one or more R^{13A}, and R^{13A} is as defined in claim 1; or R¹³ is independently selected from halogen, e.g., fluorine or chlorine.

7. The compound or the pharmaceutically acceptable salt thereof according to any of claims 1-6, wherein L₂ is selected from the group consisting of 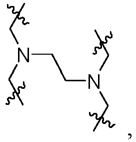 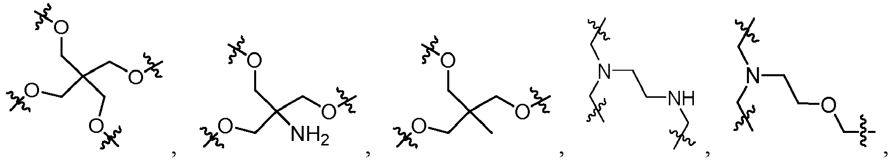 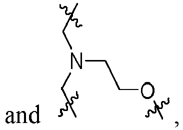 preferably

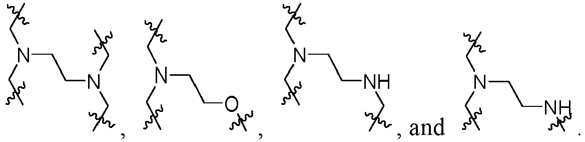

8. The compound or the pharmaceutically acceptable salt thereof according to claim 1, being selected from the group consisting of:

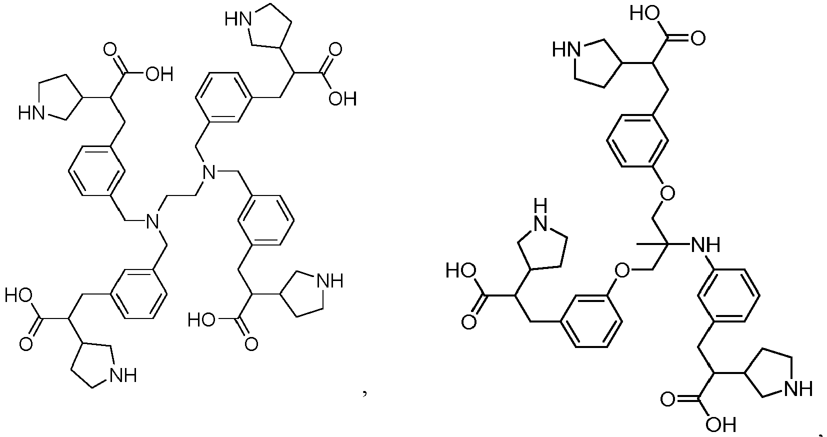

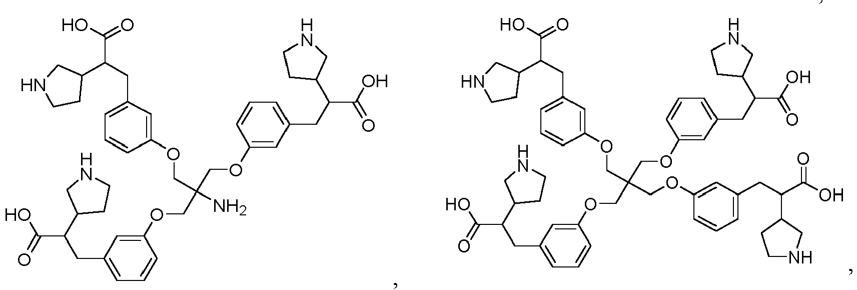

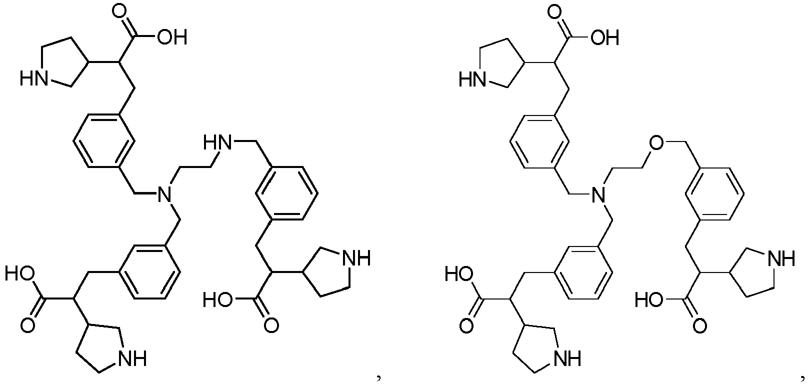

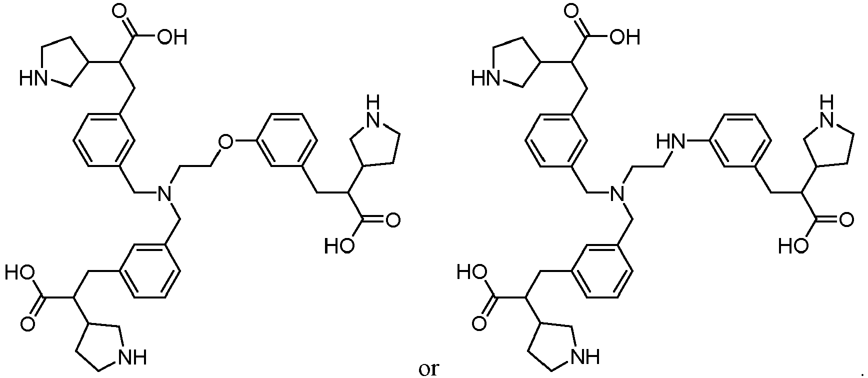

9. The compound or the pharmaceutically acceptable salt thereof according to claim 1, being selected from the group consisting of:

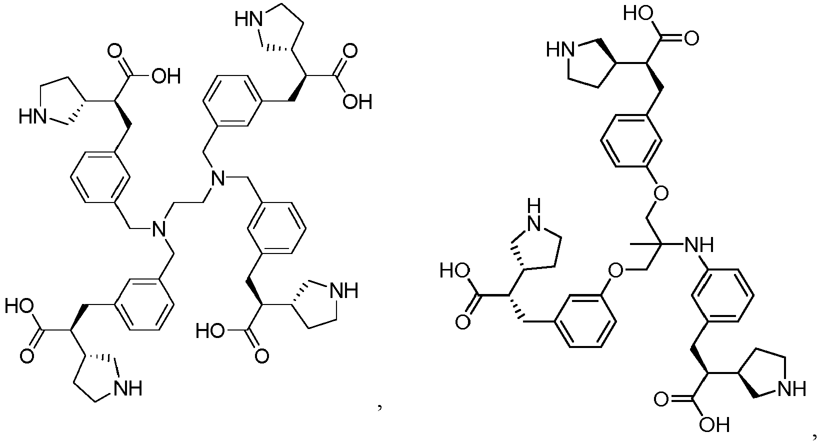

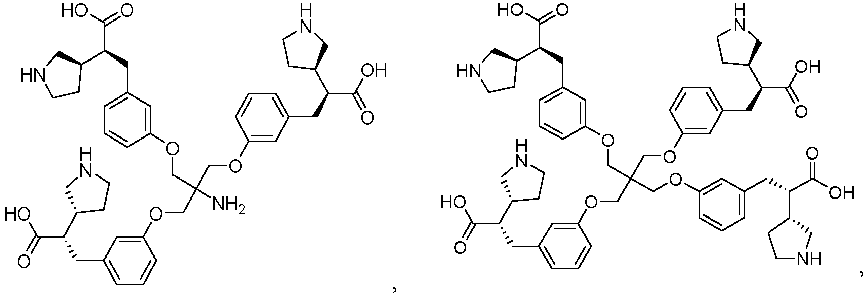

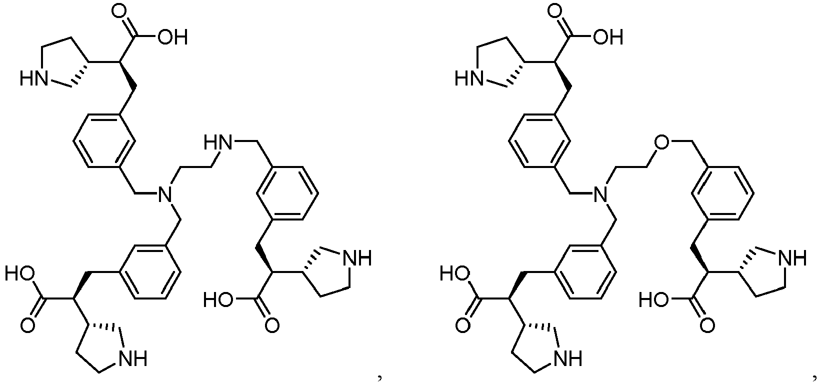

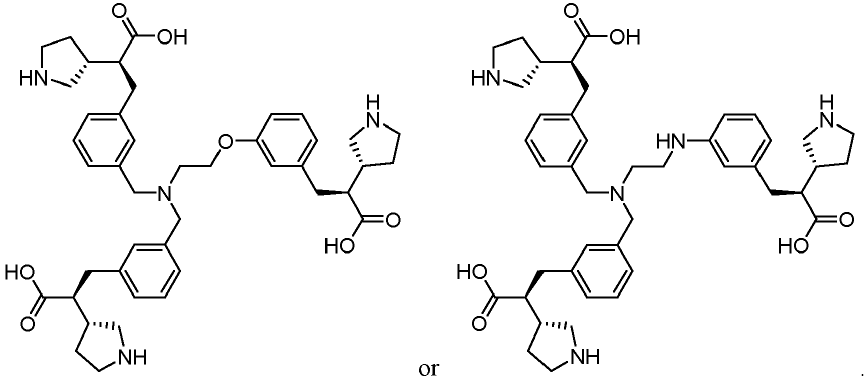

10. An isotopically substituted form of the compound or the pharmaceutically acceptable salt thereof according to any of claims 1-9, wherein preferably, the isotopically substituted form is a deuterated form.

11. A pharmaceutical composition, comprising a therapeutically effective amount of at least one of the compound or the pharmaceutically acceptable salt thereof according to any of claims 1-9 or the isotopically substituted form according to claim 10, and a pharmaceutically acceptable excipient.

12. A method for preventing and/or treating a disease or disorder associated with elevated plasma levels of LP(a), by administering to the patient a therapeutically effective amount of the compound or the pharmaceutically acceptable salt thereof according to any of claims 1-9, or the isotopically substituted form according to claim 10, or the pharmaceutical composition according to claim 11.

13. A method for preventing and/or treating a patient with a cardiovascular disease, by administering to the patient a therapeutically effective amount of the compound or the pharmaceutically acceptable salt thereof according to any of claims 1-9, or the isotopically substituted form according to claim 10, or the pharmaceutical composition according to claim 11.

14. Use of the compound or the pharmaceutically acceptable salt thereof according to any of claims 1-9, or the isotopically substituted form according to claim 10, or the pharmaceutical composition according to claim 11 in the preparation of a medicament for preventing and/or treating a disease or disorder associated with elevated plasma levels of LP(a).

15. Use of the compound or the pharmaceutically acceptable salt thereof according to any of claims 1-9, or the isotopically substituted form according to claim 10, or the pharmaceutical composition according to claim 11 in the preparation of a medicament for preventing and/or treating a cardiovascular disease.
